(19) Europäisches Patentamt
European Patent Office
Office européen des brevets

(11) EP 2 643 687 B1

(12) **EUROPEAN PATENT SPECIFICATION**

(45) Date of publication and mention of the grant of the patent:
**05.11.2014 Bulletin 2014/45**

(21) Application number: **12794332.2**

(22) Date of filing: **29.11.2012**

(51) Int Cl.:
***G01N 30/90*** *(2006.01)* ***C07D 233/54*** *(2006.01)*
***C07D 213/20*** *(2006.01)*

(86) International application number:
**PCT/EP2012/073997**

(87) International publication number:
**WO 2013/079613 (06.06.2013 Gazette 2013/23)**

(54) **Improved planar chromatography**

Verbesserte planare Chromatographie

Chromatographie de surface améliorée

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.11.2011 EP 11290546**
**29.11.2011 EP 11290545**
**29.11.2011 EP 11290547**
**29.11.2011 EP 11290548**

(43) Date of publication of application:
**02.10.2013 Bulletin 2013/40**

(73) Proprietors:
- **Total Research & Technology Feluy**
  **7181 Seneffe (BE)**
- **Centre National de la Recherche Scientifique (CNRS)**
  **75016 Paris (FR)**

(72) Inventors:
- **LAVASTRE, Olivier**
  **F-35490 Gahard (FR)**
- **FOUYER, Kevin**
  **F-68100 Mulhouse (FR)**

(74) Representative: **Bounaga, Sakina et al**
**De Clercq & Partners**
**Edgard Gevaertdreef 10a**
**9830 Sint-Martens-Latem (BE)**

(56) References cited:


- **MAKSIMOVA E F ET AL: "Methacrylate-based monolithic layers for planar chromatography of polymers", JOURNAL OF CHROMATOGRAPHY, vol. 1218, no. 17, 29 April 2011 (2011-04-29), pages 2425-2431, XP028188183, ISSN: 0021-9673, DOI: 10.1016/J.CHROMA.2010.12.061 [retrieved on 2010-12-21]**
- **DANDAN HAN ET AL: "Application of ionic liquids as mobile phase additives and surface-bonded stationary phase in liquid chromatography", KOREAN JOURNAL OF CHEMICAL ENGINEERING, vol. 26, no. 5, 1 September 2009 (2009-09-01), pages 1353-1358, XP055024405, ISSN: 0256-1115, DOI: 10.1007/s11814-009-0209-4**
- **WENTAO BI ET AL: "Comparison of Different Silica-Based Imidazolium Stationary Phases for LC in Separation of Alkaloids", CHROMATOGRAPHIA ; AN INTERNATIONAL JOURNAL FOR RAPID COMMUNICATION IN CHROMATOGRAPHY, ELECTROPHORESIS AND ASSOCIATED TECHNIQUES, VIEWEG VERLAG, WI, vol. 71, no. 1-2, 30 October 2009 (2009-10-30), pages 25-30, XP019765346, ISSN: 1612-1112**
- **AULER L M L A ET AL: "New stationary phase for anion-exchange chromatography", JOURNAL OF CHROMATOGRAPHY, ELSEVIER SCIENCE PUBLISHERS B.V, NL, vol. 1073, no. 1-2, 6 May 2005 (2005-05-06), pages 147-153, XP027723235, ISSN: 0021-9673 [retrieved on 2005-05-06]**
- **VALKENBERG M H: "Immobilisation of ionic liquids on solid supports", GREEN CHEMISTRY, vol. 4, 2002, pages 88-93, XP009158382, DOI: 10.1039/b107946h**


Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 643 687 B1

## Description

### Field of the invention

The present invention is in the field of chromatography and polymers technology.

### Background of the invention

Chromatography is a technique used for separating complex mixtures into their components. Chromatography can be described as a separation process based on difference in the rate at which the components of a mixture move through a chromatographic bed. During this process, the analytes partition between a moving phase called the mobile phase and a non-moving phase called the stationary phase. The chromatographic bed will typically include a plurality of porous, micro-porous, or nonporous particles. In some chromatographic systems, such as High Performance Liquid Chromatography (HPLC), the chromatographic bed may be packed into the interior of a column. Conversely, in other chromatographic systems, such as Thin Layer Chromatography (TLC) and Overpressured Layer Chromatography (OPLC), the chromatographic bed may be dispersed on a sample plate.

The most widely spread method for characterizing polymers is Gel Permeation Chromatography (GPC). The sample to be characterized is introduced into the chromatography column and the method allows the determination of its number average molecular weight (Mn), its weight average molecular weight (Mw), and its polydispersity index (PDI) defined as the ratio Mw/Mn of the weight average molecular weight over the number average molecular weight. This method suffers from the disadvantage that samples having a very small molecular weight pass through the chromatography column without leaving any trace. In addition the method only allows the characterization of one sample at a time.

There remains a need to provide improved chromatography method for the separation of polymers.

### Summary of the invention

It is an object of the present invention to increase the efficiency of chromatography, preferably for the separation and/or characterization of polymers, such as polymers comprising additives like antioxidant, pigments, antiacids, antistatic agents, fluidifying agents, or plastifying agents. The method is particularly advantageous when additives are not detected by GPC. It is another object of the present invention to increase the resolution of a planar chromatography for the characterization of polymers. It is also an object of the present invention to optimize separation and detection in a chromatographic process. It is also an object of the present invention to separate polymers having different molecular weights or to analyze polymers having multimodal molecular weight distributions. It is a further aim of the present invention to achieve the characterization without prior purification of the polymer samples. It is yet another aim of the present invention to carry out simultaneous characterization of several polymer samples.

The inventors have now discovered that these objects can be met either individually or in any combination by the compositions of the present invention. In accordance with the present invention, the foregoing objectives are realized as defined in the independent claims. Preferred embodiments are defined in the dependent claims.

According to a first aspect, the invention provides a composition for use in chromatography which comprises a substrate impregnated with an ionic liquid of formula (I) or (II):

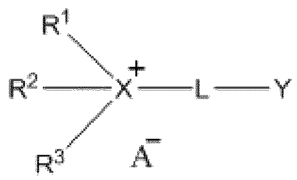

(I)

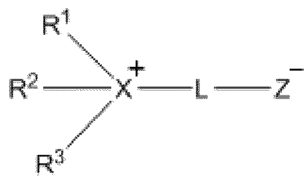

(II)

wherein

- **X** is N, P, or Al;

- **R**[1], **R**[2], and **R**[3] are each independently a group selected from $C_{1-10}$alkyl or $C_{6-12}$aryl; each group being optionally substituted with one or more substituents each independently selected from halo, $C_{1-6}$alkyl, $C_{1-6}$alkoxy, cyano, polyhalo$C_{1-6}$alkoxy, $C_{3-7}$cycloalkyl, $C_{6-12}$aryl, oxo, and Het;

**or**

**X** is N, and $\mathbf{R^1}$ is hydrogen or is not present, **and** $\mathbf{R^2}$, and $\mathbf{R^3}$, together with the atom **X** to which they are attached to, form a ring selected from pyridinium, imidazolium, pyrazolium, thiazolium, triazolium, pyrrolium, indolium, tetrazolium, pyrrolidinium, morpholinium pyrimidinium, pyrazinium, pyridazinium, piperazinium, and piperidinium; each optionally substituted with one, two, or three substituents selected from $C_{1-12}$alkyl and $C_{1-12}$alkoxy;

**L** is $C_{1-12}$alkylene, $C_{3-7}$cycloalkylene, $C_{6-12}$arylene; each of $C_{1-6}$alkylene, $C_{3-7}$cycloalkylene, and $C_{6-12}$arylene, optionally containing 1 to 4 heteroatoms each independently selected from nitrogen, oxygen and sulfur; and each of $C_{1-6}$alkylene, $C_{3-7}$cycloalkylene, and $C_{6-12}$arylene, being optionally substituted one or more substituents each independently selected from $C_{1-6}$alkyl and oxo;

**Y** is hydrogen, halo, $C_{1-10}$alkyl, $C_{6-12}$aryl, $-OR^4$, $-COOR^4$, $-CONR^{5a}R^{5b}$, $-NR^{5a}R^{5b}$, $-SR^6$, - $SO_2R^7$, $-SiR^8{}_3$, $-OP(O)(OH)_2$, epoxy, or Het; each group being optionally substituted with one or more substituents each independently selected from halo, OH, $-OR^4$,$-COR^4$, $C_{1-6}$alkyl; $C_{6-12}$aryl, $C_{1-6}$alkoxy, oxo, halo$C_{1-6}$alkyl, and halo$C_{1-6}$alkoxy;

**Z** is a carboxylate ($-CO_2$), a sulfonate ($-SO_3$), a sulfinate ($-SO_2$), a phosphorus based anion (such as phosphonate, phosphite, phosphate and the like), an ester monosulfate ($-OSO_3$), or a sulfonamidate ($-NHSO_2$);

**A** is $[PF_6]$, $[AsF_6]$, $[SbF_6]$, $[N(SO_2CF_3)_2]$, $[BF_4]$, $[CF_3SO_3]$, $[CF_3CO_2]$, $[CH_3CO_2]$, $[CF_3SO_2]$, $[NO_2]$, $[NO_3]$, $[ClO_4]$, [Cl], [Br], [I], [HO-CO-O]; and $[AlR^{10}{}_{4-n}R^{11}{}_n]$;

$\mathbf{R^4}$ is hydrogen; or a group selected from $C_{1-6}$alkyl; $C_{6-12}$aryl; Het; and $C_{3-7}$cycloalkyl; each group being optionally substituted with one or more substituents each independently selected from $C_{1-6}$alkyl; $C_{3-7}$cycloalkyl, $C_{6-12}$aryl, halo, $C_{1-6}$alkoxy, oxo and Het;

$\mathbf{R^{5a}}$ and $\mathbf{R^{5b}}$ are, each independently, selected from hydrogen, $C_{1-6}$alkyl, and $C_{6-12}$aryl$C_{1-6}$alkyl;

$\mathbf{R^6}$ is hydrogen, $C_{1-6}$alkyl, $C_{6-12}$aryl, $C_{3-7}$cycloalkyl, or $-C(=O)R^9$;

$\mathbf{R^7}$ is hydrogen, $C_{1-6}$alkyl, or $C_{3-7}$cycloalkyl;

$\mathbf{R^8}$ is hydrogen, $C_{1-6}$alkyl optionally substituted with halo, $C_{1-6}$alkoxy, $C_{3-7}$cycloalkyl, or $C_{6-12}$aryl; $C_{3-7}$cycloalkyl; or - $OR^4$;

$\mathbf{R^9}$ is $C_{1-12}$alkyl;

$\mathbf{R^{10}}$ is Cl, Br, F or I or $C_{1-12}$alkyl;

$\mathbf{R^{11}}$ is F, Cl, Br, or I;

**n** is an integer from 0 to 4;

$\mathbf{C_{6-12}aryl}$ as a group or part of a group is phenyl, naphthyl, indanyl, or 1,2,3,4-tetrahydronaphthyl, each of which may be optionally substituted with one, two or three substituents selected from halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, mono- or di$C_{1-6}$alkylaminocarbonyl, azido, mercapto;

**Het** as a group or part of a group is a 5 or 6 membered saturated, partially unsaturated or completely unsaturated heterocyclic ring containing 1 to 4 heteroatoms each independently selected from nitrogen, oxygen and sulfur, said heterocyclic ring being optionally condensed with a benzene ring, and wherein the group Het as a whole may be optionally substituted with one, two or three substituents each independently selected from the group consisting of halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, and mono- or di$C_{1-6}$alkylaminocarbonyl.

It should be noted in all applicable embodiments that in the definition of $\mathbf{R^1}$, such substituent is hydrogen when $\mathbf{R^2}$, and $\mathbf{R^3}$, together with the atom **X,** i.e. nitrogen, form a non-aromatic ring and the nitrogen atom bears no double bond.

It should be further noted in all applicable embodiments that in the definition of $\mathbf{R^1}$, such substituent is not present when $\mathbf{R^2}$, and $\mathbf{R^3}$, together with the atom **X,** i.e. nitrogen, form an aromatic ring.

In a second aspect, the present invention provides the use of a composition according to the first aspect of the invention as a stationary phase in chromatography.

In a third aspect, the present invention provides a method of performing planar chromatography characterized in that the planar stationary phase is a composition according to the first aspect of the invention.

The invention also encompasses a method for analyzing, separating, or characterizing one or more samples, in particular polymer samples, comprising loading one or more samples onto a stationary phase and while retaining the sample on the stationary phase, subjecting the sample to chromatography (chromatographic separation) using a mobile phase; wherein the stationary phase is impregnated with a compound of formula (I) or (II) as described herein. In a preferred embodiment, said stationary phase is a planar stationary phase.

Preferably, the present invention relates to a method of performing planar chromatography in a method for analyzing, separating, or characterizing polymers selected from polyolefins, polyamides, polycarbonates, poly(hydroxy carboxylic acid), polystyrenes, polyesters, polyethylene terephthalate (PET), polybutylene terephthalate (PBT), polymethylmethacrylate (PMMA), poly(methyl acrylate) (PMA), vinyl polymers, or blends thereof, wherein the planar stationary phase is a substrate impregnated with an ionic liquid of formula (I), (II), (III) or (IV),

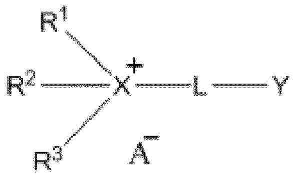

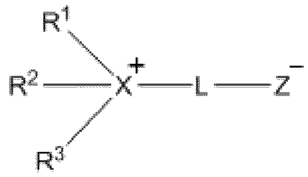

(I) (II)

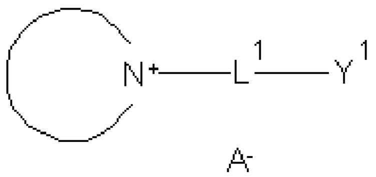

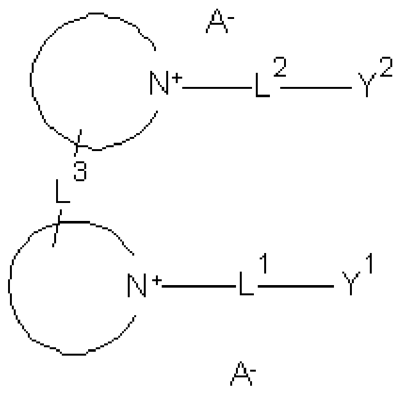

(III) (IV)

wherein

**X** is N, P, or Al;

$\mathbf{R^1}$**,** $\mathbf{R^2}$**,** and $\mathbf{R^3}$ are each independently a group selected from $C_{1-10}$alkyl or $C_{6-12}$aryl; each group being optionally substituted with one or more substituents each independently selected from halo, $C_{1-6}$alkyl, $C_{1-6}$alkoxy, cyano, polyhalo$C_{1-6}$alkoxy, $C_{3-7}$cycloalkyl, $C_{6-12}$aryl, oxo, and Het;

**or**

**X** is N, and $\mathbf{R^1}$ is hydrogen or is not present, and $\mathbf{R^2}$, and $\mathbf{R^3}$, together with the atom **X** to which they are attached to, form a ring selected from pyridinium, imidazolium, pyrazolium, thiazolium, triazolium, pyrrolium, indolium, tetrazolium, pyrrolidinium, morpholinium pyrimidinium, pyrazinium, pyridazinium, piperazinium, and piperidinium; each optionally substituted with one, two, or three substituents selected from $C_{1-12}$alkyl and $C_{1-12}$alkoxy;

each **ring** comprising the nitrogen atom is independently selected from the group consisting of pyridinium, pyrazinium, pyrrolium, imidazolium, pyrazolium, thiazolium, triazolium, indolium, tetrazolium, pyrimidinium, pyridazinium, piperazinium, and piperidinium; each group being optionally substituted with one, two, or three substituents selected from $C_{1-12}$alkyl and $C_{1-12}$alkoxy;

**L** is $C_{1-12}$alkylene, $C_{3-7}$cycloalkylene, $C_{6-12}$arylene; each of $C_{1-6}$alkylene, $C_{3-7}$cycloalkylene, and $C_{6-12}$arylene, optionally containing 1 to 4 heteroatoms each independently selected from nitrogen, oxygen and sulfur; and each of $C_{1-6}$alkylene, $C_{3-7}$cycloalkylene, and $C_{6-12}$arylene, being optionally substituted one or more substituents each independently selected from $C_{1-6}$alkyl and oxo;

$\mathbf{L^1}$ is $C_{1-12}$alkylene;

$\mathbf{L^2}$ is $C_{1-12}$alkylene;

$\mathbf{L^3}$ is a single bond or $C_{1-12}$alkylene; wherein one carbon of the $C_{1-12}$alkylene is optionally replaced by one or more heteroatoms;

**Y** is hydrogen, halo, $C_{1-10}$alkyl, $C_{6-12}$aryl, $-OR^4$, $-COOR^4$, $-CONR^{5a}R^{5b}$, $-NR^{5a}R^{5b}$, $-SR^6$, - $SO_2R^7$, $-SiR^8{}_3$, $-OP(O)(OH)_2$, epoxy, or Het; each group being optionally substituted with one or more substituents each independently selected from halo, OH, $-OR^4$,$-COR^4$, $C_{1-6}$alkyl; $C_{6-12}$aryl, $C_{1-6}$alkoxy, oxo, halo$C_{1-6}$alkyl, and halo$C_{1-6}$alkoxy;

$\mathbf{Y^1}$ is $C_{6-12}$aryl substituted with one, two, or three substituents each independently selected from the group consisting of halo, halo$C_{1-6}$alkyl, and halo$C_{1-6}$alkoxy;

$\mathbf{Y^2}$ is $C_{6-12}$aryl substituted with one, two, or three substituents each independently selected from the group consisting of halo, halo$C_{1-6}$alkyl, and halo$C_{1-6}$alkoxy;

**Z** is a carboxylate ($-CO_2$), a sulfonate ($-SO_3$), a sulfinate ($-SO_2$), a phosphorus based anion (such as phosphonate, phosphite, phosphate and the like), an ester monosulfate ($-OSO_3$), or a sulfonamidate ($-NHSO_2$);

**A** is [Br], $[PF_6]$, $[BF_4]$, $[AsF_6]$, $[SbF_6]$, $[N(SO_2CF_3)_2]$, $[CF_3SO_3]$, $[CF_3CO_2]$, $[CH_3CO_2]$, $[CF_3SO_2]$, $[NO_2]$, $[NO_3]$, $[ClO_4]$, [Cl], [I], [HO-CO-O]; and $[AlR^{10}{}_{4-n}R^{11}{}_n]$;

$\mathbf{R^4}$ is hydrogen; or a group selected from $C_{1-6}$alkyl; $C_{6-12}$aryl; Het; and $C_{3-7}$cycloalkyl; each group being optionally substituted with one or more substituents each independently selected from $C_{1-6}$alkyl; $C_{3-7}$cycloalkyl, $C_{6-12}$aryl, halo, $C_{1-6}$alkoxy, oxo and Het;

$\mathbf{R^{5a}}$ and $\mathbf{R^{5b}}$ are, each independently, selected from hydrogen, $C_{1-6}$alkyl, and $C_6$-$_{12}$aryl$C_{1-6}$alkyl;

$\mathbf{R^6}$ is hydrogen, $C_{1-6}$alkyl, $C_{6-12}$aryl, $C_{3-7}$cycloalkyl, or $-C(=O)R^9$;

$\mathbf{R^7}$ is hydrogen, $C_{1-6}$alkyl, or $C_{3-7}$cycloalkyl;

$\mathbf{R^8}$ is hydrogen, $C_{1-6}$alkyl optionally substituted with halo, $C_{1-6}$alkoxy, $C_{3-7}$cycloalkyl, or $C_{6-12}$aryl; $C_{3-7}$cycloalkyl; or $-OR^4$;

$\mathbf{R^9}$ is $C_{1-12}$alkyl;

$\mathbf{R^{10}}$ is Cl, Br, F or I or $C_{1-12}$alkyl;

$\mathbf{R^{11}}$ is F, Cl, Br, or I;

**n** is an integer from 0 to 4;

$\mathbf{C_{6-12}}$**aryl** as a group or part of a group is phenyl, naphthyl, indanyl, or 1,2,3,4-tetrahydronaphthyl, each of which may be optionally substituted with one, two or three substituents selected from halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, mono- or di$C_{1-6}$alkylaminocarbonyl, azido, mercapto;

**Het** as a group or part of a group is a 5 or 6 membered saturated, partially unsaturated or completely unsaturated heterocyclic ring containing 1 to 4 heteroatoms each independently selected from nitrogen, oxygen and sulfur, said heterocyclic ring being optionally condensed with a benzene ring, and wherein the group Het as a whole may be optionally substituted with one, two or three substituents each independently selected from the group consisting of halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, and mono- or di$C_{1-6}$alkylaminocarbonyl.

More preferably the polymer is a polyolefin, polystyrene or polylactic acid, preferably the polymer is selected from polyethylene, polypropylene, or polylactic acid.

A major advantage of the present method is that it is inexpensive and simple to carry out. Furthermore, the separation can be carried out one- or multidimensionally, meaning that the resolution of the separation can be matched to the separation problem.

The independent and dependent claims set out particular and preferred features of the invention. Features from the dependent claims may be combined with features of the independent or other dependent claims as appropriate.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

**Brief description of the figures**

Figure 1 represents pictures of two TLC plates on which polypropylene has been eluted. Figure 1A shows a plate wherein the stationary phase was not impregnated with a liquid ionic, and Figure 1B shows a plate wherein the stationary phase was impregnated with a ionic liquid.

**Description of the invention**

Before the present method and products of the invention are described, it is to be understood that this invention is not limited to particular methods, components, products or combinations described, as such methods, components, products and combinations may, of course, vary. It is also to be understood that the terminology used herein is not intended to be limiting, since the scope of the present invention will be limited only by the appended claims.

In a first embodiment, the present invention relates to a composition for use in chromatography comprising a substrate impregnated with an ionic liquid of formula (I) or (II):

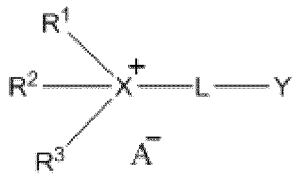
(I)

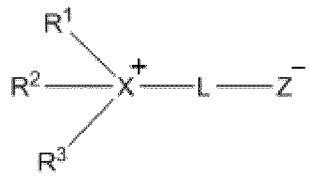
(II)

wherein

**X** is N, P, or Al;

**$R^1$**, **$R^2$**, and **$R^3$** are each independently a group selected from $C_{1-10}$alkyl or $C_{6-12}$aryl; each group being optionally substituted with one or more substituents each independently selected from halo, $C_{1-6}$alkyl, $C_{1-6}$alkoxy, cyano, polyhalo$C_{1-6}$alkoxy, $C_{3-7}$cycloalkyl, $C_{6-12}$aryl, oxo or with Het;

or **X** is N, and **$R^1$**, **$R^2$**, and **$R^3$**, together with the atom **X** to which they are attached to, form a ring selected from pyridinium, imidazolium, pyrazolium, thiazolium, triazolium, pyrrolium, indolium, tetrazolium, pyrimidinium, pyrazinium, pyridazinium, piperazinium, pyrrolidinium, morpholinium and piperidinium;

**L** is $C_{1-6}$alkylene, $C_{3-7}$cycloalkylene, $C_{6-12}$arylene; each of $C_{1-6}$alkylene, $C_{3-7}$cycloalkylene, and $C_{6-12}$arylene, op-

tionally containing 1 to 4 heteroatoms each independently selected from nitrogen, oxygen and sulfur; and each of $C_{1-6}$alkylene, $C_{3-7}$cycloalkylene, and $C_{6-12}$arylene, being optionally substituted with one or more substituents each independently selected from $C_{1-6}$alkyl or oxo;

**Y** is hydrogen, halo, or a group selected from $C_{1-10}$alkyl, $C_{6-12}$aryl, $-OR^4$, $-COOR^4$, $-CONR^{5a}R^{5b}$, $-NR^{5a}R^{5b}$, $-SR^6$, $-SO_2R^7$, $-SiR^8_3$, $-OP(O)(OH)_2$, epoxy, or Het; each group being optionally substituted with one or more substituents each independently selected from halo, OH, $-OR^4$, $-COR^4$, $C_{1-6}$alkyl; $C_{6-12}$aryl, $C_{1-6}$alkoxy, or oxo;

**Z$^-$** is a carboxylate, a sulfonate, a sulfinate, a phosphorus based anion, an ester monosulfate, or a sulfonamidate;

**A** is $[PF_6^-]$, $[AsF_6^-]$, $[SbF_6^-]$, $[N(SO_2CF_3)_2^-]$, $[BF_4^-]$, $[CF_3SO_3^-]$, $[CF_3CO_2^-]$, $[CH_3CO_2^-]$, $[CF_3SO_2^-]$, $[NO_2^-]$, $[NO_3^-]$, $[ClO_4^-]$, $[Cl^-]$, $[Br^-]$, $[I^-]$, [HO-CO-O-]; and $[AlR^{10}_{4-n}R^{11}_n{}^-]$;

**R$^4$** is hydrogen; or a group selected from $C_{1-6}$alkyl; $C_{6-12}$aryl; Het; or $C_{3-7}$cycloalkyl; each group being optionally substituted with one or more substituents each independently selected from $C_{1-6}$alkyl; $C_{3-7}$cycloalkyl, $C_{6-12}$aryl, halo, $C_{1-6}$alkoxy, oxo or Het;

**R$^{5a}$** and **R$^{5b}$** are, each independently, selected from hydrogen, $C_{1-6}$alkyl, or $C_{6-12}$aryl$C_{1-6}$alkyl;

**R$^6$** is hydrogen, $C_{1-6}$alkyl, $C_{6-12}$aryl, $C_{3-7}$cycloalkyl, or $-C(O)R^9$;

**R$^7$** is hydrogen, $C_{1-6}$alkyl or $C_{3-7}$cycloalkyl;

**R$^8$** is hydrogen, $C_{1-6}$alkyl optionally substituted with halo, $C_{1-6}$alkoxy, $C_{3-7}$cycloalkyl, or $C_{6-12}$aryl; $C_{3-7}$cycloalkyl; or - $OR^4$;

**R$^9$** is $C_{1-12}$alkyl;

**R$^{10}$** is Cl, Br, F or I or $C_{1-12}$alkyl;

**R$^{11}$** is F, Cl, Br, or I;

**n** is an integer from 0 to 4;

**Het** as a group or part of a group is a 5 or 6 membered saturated, partially unsaturated or completely unsaturated heterocyclic ring containing 1 to 4 heteroatoms each independently selected from nitrogen, oxygen and sulfur, said heterocyclic ring being optionally condensed with a benzene ring, and wherein the group Het as a whole may be optionally substituted with one, two or three substituents each independently selected from the group consisting of halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, and mono- or di$C_{1-6}$alkylaminocarbonyl.

In a second embodiment relating to the composition of the first embodiment,

**X** is N, **R$^1$**, **R$^2$**, and **R$^3$**, together with the atom **X** to which they are attached to, form a ring selected from pyridinium, imidazolium, pyrazolium, thiazolium, triazolium, pyrrolium, indolium, tetrazolium, pyrimidinium, pyrazinium, pyridazinium, piperazinium, and piperidinium;

**L** is $C_{1-6}$alkylene;

**Y** is $C_{6-12}$aryl optionally substituted with one or more halo substituents such as chloro or bromo; and

**A** is $[Br^-]$, $[Cl^-]$, $[PF_6^-]$, $[AsF_6^-]$, $[SbF_6^-]$, $[N(SO_2CF_3)_2^-]$, $[BF_4^-]$, $[CF_3SO_3^-]$, $[CH_3CO_2^-]$, $[CF_3SO_2^-]$, $[NO_2^-]$, $[NO_3^-]$, $[ClO_4^-]$, $[I^-]$, and $[AlR^{10}_{4-n}R^{11}_n{}^-]$.

In a third embodiment relating to any one of the first to the second embodiments, the substrate comprises silica based normal and reverse-phase resin optionally derivatized with alkyl groups or aromatic groups, preferably silica gel 60F254.

In a fourth embodiment, the present invention relates to the use of a composition according to any one of the first to the third embodiments, as a stationary phase in chromatography.

In a fifth embodiment, the present invention relates to a method of performing planar chromatography characterized in that the planar stationary phase is a composition according to any of the first to the third embodiments above, or in that the planar stationary phase is impregnated with an ionic liquid of formula (I) or (II), as described in any one of the first to the third embodiments above.

In a sixth embodiment, the present invention relates to a method according to the fifth embodiment above, in a method for analyzing, separating, or characterizing polymers selected from polyolefins, polyamides, polycarbonates, poly(hydroxy carboxylic acid), polystyrenes, polyesters, polyethylene terephthalate (PET), polybutylene terephthalate (PBT), polymethylmethacrylate (PMMA), poly(methyl acrylate) (PMA), vinyl polymers, proteins, and polysaccharides, or blends thereof.

In a seventh embodiment, the present invention relates to a method according to any one of the fifth to the sixth embodiments above, wherein the chromatography is performed at a working temperature between 30°C and 250°C.

In an eighth embodiment, the present invention relates to a method according to any one of the fifth to the seventh embodiments above, wherein the chromatography is performed with a temperature gradient.

In a ninth embodiment, the present invention relates to the method according to the eighth embodiment above, wherein the temperature gradient is greater than, or equal to 1°C per minute.

In a tenth embodiment, the present invention relates to the method according to any one of the fifth to the ninth embodiments above, wherein the chromatography is performed using a mobile phase selected from the group comprising toluene, xylene, tetrahydrofuran, $C_4$-$C_{20}$ branched or non-branched alkane, supercritical carbon dioxide, trichlorobenzene, ethyl acetate, dimethylsulfoxide, dimethylformamide, an ionic liquid of formula (I) or (II), and mixtures thereof; optionally in the presence of a co-eluent selected from the group comprising an alcohol and an ionic liquid of formula (I) or (II).

In an eleventh embodiment, the present invention relates to the method according to any one of the fifth to the tenth embodiments above, wherein the chromatography is performed under external pressure by applying either i) a neutral gas under pressure applied to a membrane on the planar stationary phase; ii) a pneumatic pillow or metallic membrane on the planar stationary phase; or iii) a pneumatic press on the planar stationary phase.

In a twelfth embodiment, the present invention relates to the method according to any one of the fifth to the eleventh embodiments above, wherein the chromatography is subjected to a gradient elution and comprises at least two subsequent chromatography steps with progressively decreasing migration distances of the mobile phase on the planar chromatography plate, wherein said gradient elution comprises increasing the elution strength of the mobile phase between two subsequent chromatography steps, said steps being performed on the same planar chromatography plate.

In a thirteenth embodiment, the present invention relates to the method according to any one of the fifth to the twelfth embodiments above, wherein planar chromatography is selected from a Thin-layer chromatography (TLC), High Performance Thin-Layer Chromatography (HPTLC), and Overpressurized Layer Chromatography (OPLC).

In a fourteenth embodiment, the present invention relates to the method according to any one of the fifth to the thirteenth embodiments above, wherein planar chromatography is Overpressurized Layer Chromatography (OPLC).

In a fifteenth embodiment, the present invention relates to the method according to any one of the fifth to the fourteenth embodiments above, in a method for analyzing, separating, and/or characterizing polyolefins.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "chromatography" refers to a physical method of separation in which the components to be separated are distributed between two phases, one of which is stationary (stationary phase) while the other (the mobile phase) moves in a definite direction.

The term "planar chromatography" refers to a separation technique in which the stationary phase is present as or on a plane (planar stationary phase). The plane can be a paper, used as such or impregnated by a substrate as the stationary bed (paper chromatography, PC) or a layer of solid particles spread on a support e.g. a glass or metal plate.

The term "support" or "plate" or "support plate" refers to the plate that supports the stationary phase, such as the thin layer in thin-layer chromatography.

In accordance with an embodiment of the invention, a planar chromatography plate is any medium on which a planar chromatographic separation can be carried out. In general, a plate consists of a support, for example in the form of a glass plate, metal plate or foil or a plastic film which is covered or coated with the stationary phase.

The terms "chromatography step", "chromatography run", "elution step" or "separation step" are used interchangeably and refer to a step wherein the sample to be analyzed, separated and/or characterized is deposited on a stationary phase and wherein the said sample is put in conditions to migrate along at least one axis of the chromatography plate, according to at least one physical or chemical property of the said sample, such as molecular mass, electric charge, acid/basic properties, etc.

The terms "stationary phase" or "stationary bed" or "sorbent bed" or "absorbent" are used interchangeably and refer to an immobile phase or non-fluid phase employed in the chromatography method. The expression chromatographic bed or sorbent bed may be used as a general term to denote any of the different forms in which the stationary phase is used. The stationary phases used are typically the base sorbents known for chromatographic purposes. These are, for example, silica gel, aluminium oxide, cellulose, kieselguhr or other organic or inorganic polymers or organic/inorganic hybrid polymers. The base sorbents may furthermore be derivatized with functional groups which modify their separation properties. Examples thereof are RP phases, in which, for example, silica gel has been derivatized with ligands which have C8 or C18 chains (reversed phase material). Other examples are CN or diol-modified phases. Suitable common sorbent phases for planar chromatography are described in Klaus K. Unger, Packings and Stationary Phases in Chromatographic Techniques, M. Decker, New York 1990.

The term "mobile phase" refers to a fluid that migrates through or along the stationary bed, in a definite direction, carrying thereby the sample through the stationary phase. This fluid may be a liquid or a supercritical fluid. The term "eluent" is also used for the mobile phase.

The term "elution strength" or strength of a mobile phase is used to describe the affinity that a sample component will have for either the mobile phase or stationary phase.

The terms "strong mobile phase" and "weak mobile phase" are known in the art. As used herein, a "strong mobile phase" refers to a mobile phase that has a high elution strength and results in little or no retention of the sample on the stationary phase. A sample in a "strong" mobile phase will have a greater affinity for the mobile phase than the stationary phase, resulting in little or no retention of the sample on the stationary phase and a shorter elution time.

As used herein, a "weak mobile phase" refers to a mobile phase that has a low elution strength and results in higher amount of retention of the sample on the stationary phase relative to a strong mobile phase. Contrary to a sample in a strong mobile phase, a sample provided in a "weak" mobile phase will have less affinity for the mobile phase than the stationary phase, resulting in sample components being strongly retained on the stationary phase and a longer elution time.

The term "thin-layer chromatography" or TLC refers to a chromatography carried out in a thin layer of adsorbent spread on a support e.g. a glass or metal plate.

The term "spot" in chromatography refers to a zone on the planar chromatography plate of approximately circular appearance.

The term "temperature gradient" refers to the procedure in which the temperature of the stationary phase, the mobile phase, the plate, or all, is changed systematically during a part or the whole of the separation or chromatography process.

The term "gradient elution" or "solvent gradient" or "gradient mobile phase" in the field of chromatography refers to a procedure in which the composition of the mobile phase is changed continuously or stepwise during the elution process. Preferably the composition of the mobile phase is changed stepwise. Preferably the composition of the mobile phase is changed in terms of elution strength.

The term "stepwise elution" in chromatography refers to an elution process in which the composition of the mobile phase is changed in steps during a single chromatographic run or between two subsequent chromatography steps.

The term "normal-phase chromatography" refers to an elution procedure in which the stationary phase is more polar then the mobile phase.

The term "reversed-phase chromatography" refers to an elution procedure used in chromatography in which the mobile phase is significantly more polar then the stationary phase, e.g. a microporous silica-based material with chemically bonded alkyl chains.

The term "retardation factor, $\boldsymbol{R_F}$" in planar chromatography refers to the ratio of the distance travelled by the centre of the spot (b) to the distance simultaneously travelled by the mobile phase (a): $R_F = \frac{b}{a}$ By definition the $\boldsymbol{R_F}$ values are always less than unity.

The term "programmed-pressure chromatography" or "pressure programming" refers to a procedure in which the inlet pressure of the mobile phase is changed systematically during a part or whole of the separation.

Whenever the term "substituted" is used in defining the compounds of the present invention, it is meant to indicate that one or more hydrogens on the atom indicated in the expression using "substituted" is replaced with a selection from the indicated group, provided that the indicated atom's normal valency is not exceeded, and that the substitution results in a chemically stable compound, i.e. a compound that is sufficiently robust to survive isolation to a

useful degree of purity from a reaction mixture.

As used herein, the term "halo" or "halogen" as a group or part of a group is generic for fluoro, chloro, bromo or iodo.

The term "$C_{1-6}$alkyl" as a group or part of a group defines straight and branched chained saturated hydrocarbon radicals having from 1 to 6 carbon atoms such as, for example, methyl, ethyl, propyl, butyl, 2-methyl-propyl, pentyl, hexyl, 2-methylbutyl, 3-methylpentyl and the like.

The term "$C_{1-12}$alkyl" as a group or part of a group defines straight and branched chained saturated hydrocarbon radicals having from 1 to 12 carbon atoms, such as, those defined for $C_{1-6}$alkyl and heptyl, octyl, 2-methyl-hetyl, 3-ethylhexyl, nonyl, decyl, undecanyl, dodecanyl, and the like.

The term "$C_{3-7}$cycloalkyl" as a group or part of a group is generic to cyclopropyl, cyclobutyl, cyclopentyl, cyclohexyl, or cycloheptyl.

The term "$C_{5-6}$cycloalkyl" as a group or part of a group is generic to cyclopentyl or cyclohexyl.

The term "$C_{1-12}$alkylene" as a group or part of a group defines bivalent straight and branched chained saturated hydrocarbon radicals having from 1 to 6 carbon atoms such as, for example, methylene, ethan-1,2-diyl, propan-1,3-diyl, propan-1,2-diyl, butan-1,4-diyl, pentan-1,5-diyl, hexan-1,6-diyl, 2-methylbutan-1,4-diyl, 3-methylpentan-1,5-diyl, octan-1,8-diyl, undecan-1,9-diyl, dodecan-1,12-diyl, and the like.

The term "$C_{3-7}$cycloalkylene" as a group or part of a group defines a bivalent saturated hydrocarbon ring having from 3 to 7 carbon atoms.

The term "$C_{6-12}$aryl", as a group or part of a group, refers to a polyunsaturated, aromatic hydrocarbyl group having a single ring (i.e. phenyl) or multiple aromatic rings fused together (e.g. naphthalene), or linked covalently, typically containing 6 to 12 atoms; wherein at least one ring is aromatic. Non-limiting examples of $C_{6-12}$aryl comprise phenyl, biphenylyl, biphenylenyl, indanyl, or 1,2,3,4-tetrahydronaphthyl, or 1-or 2-naphthanelyl, each of which may be optionally substituted with one, two or three substituents selected from halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, mono- or di$C_{1-6}$alkylaminocarbonyl, azido, mercapto.

The term "$C_{6-12}$arylene" as a group or part of a group defines a bivalent aromatic hydrocarbon ring having 6 to 12 carbon atoms.

The term "$C_{1-6}$alkoxy" or "$C_{1-6}$alkyloxy" as used herein refers to a radical having the Formula $-OR^a$ wherein $R^a$ is $C_{1-6}$alkyl as defined herein. Non-limiting examples of suitable alkoxy include methoxy, ethoxy, propoxy, isopropoxy, butoxy, isobutoxy, sec-butoxy, tert-butoxy, pentyloxy and hexyloxy.

The term "halo$C_{1-6}$alkyl" as used herein refers to an $C_{1-6}$alkyl as defined herein wherein one or more hydrogens are replaced with a halogen, preferably, chloro or fluoro atoms, more preferably fluoro atoms. Examples of such halo$C_{1-6}$alkyl radicals include chloromethyl, 1-bromoethyl, fluoromethyl, difluoromethyl, trifluoromethyl, 1,1,1-trifluoroethyl, 1-bromopropyl, 2-fluorobutyl, 3,4-difluoropentyl, 3,4,4-trifluoropentyl, 1,1,1-trifluorohexyl, and the like.

The term "halo$C_{1-6}$alkoxy" or "halo$C_{1-6}$alkyloxy" as used herein refers to a radical having the Formula $-OR^b$ wherein $R^b$ is halo$C_{1-6}$alkyl as defined herein. Non-limiting examples of suitable alkoxys include chloromethoxy, 1-bromoethoxy, fluoromethoxy, difluoromethoxy, trifluoromethoxy, 1,1,1-trifluoroethoxy, 1-bromopropoxy, 2-fluorobutoxy, 3,4-difluoropentyloxy, 3,4,4-trifluoropentyloxy, 1,1,1-trifluorohexyloxy, and the like.

The term "oxo" refers to the group =O and forms a carbonyl moiety when attached to a carbon atom, a sulfoxide moiety when attached to a sulfur atom and a sulfonyl moiety when two of said terms are attached to a sulfur atom. Whenever a ring or ring system is substituted with an oxo group, the carbon atom to which the oxo is linked is a saturated carbon.

The present invention also relates to a composition for use in chromatography comprising a substrate impregnated with an ionic liquid of formula (I), (II), (III) or (IV);

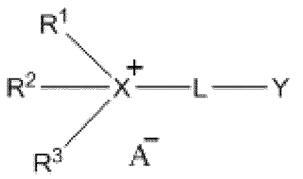

(I)

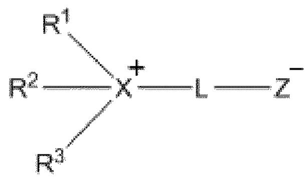

(II)

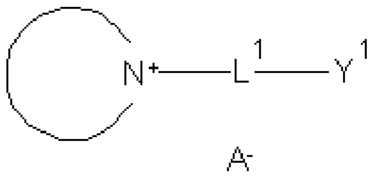

(III)

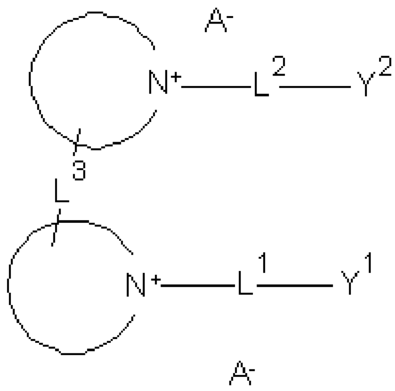

(IV)

wherein

**X** is N, P, or Al;

**R$^1$**, **R$^2$**, and **R$^3$** are each independently a group selected from $C_{1-10}$alkyl or $C_{6-12}$aryl; each group being optionally substituted with one or more substituents each independently selected from halo, $C_{1-6}$alkyl, $C_{1-6}$alkoxy, cyano, polyhalo$C_{1-6}$alkoxy, $C_{3-7}$cycloalkyl, $C_{6-12}$aryl, oxo, and Het;

or

**X** is N, and **R$^1$** is hydrogen or is not present, **and R$^2$**, and **R$^3$**, together with the atom **X** to which they are attached to, form a ring selected from pyridinium, imidazolium, pyrazolium, thiazolium, triazolium, pyrrolium, indolium, tetrazolium, pyrrolidinium, morpholinium pyrimidinium, pyrazinium, pyridazinium, piperazinium, and piperidinium; each optionally substituted with one, two, or three substituents selected from $C_{1-12}$alkyl and $C_{1-12}$alkoxy;

each **ring** comprising the nitrogen atom is independently selected from the group consisting of pyridinium, pyrazinium, pyrrolium, imidazolium, pyrazolium, thiazolium, triazolium, indolium, tetrazolium, pyrimidinium, pyridazinium, piperazinium, and piperidinium; each group being optionally substituted with one, two, or three substituents selected from $C_{1-12}$alkyl and $C_{1-12}$alkoxy;

**L** is $C_{1-12}$alkylene, $C_{3-7}$cycloalkylene, $C_{6-12}$arylene; each of $C_{1-6}$alkylene, $C_{3-7}$cycloalkylene, and $C_{6-12}$arylene, optionally containing 1 to 4 heteroatoms each independently selected from nitrogen, oxygen and sulfur; and each of $C_{1-6}$alkylene, $C_{3-7}$cycloalkylene, and $C_{6-12}$arylene, being optionally substituted one or more substituents each independently selected from $C_{1-6}$alkyl and oxo;

**L$^1$** is $C_{1-12}$alkylene; preferably $C_{1-6}$alkylene; more preferably $C_{1-2}$alkylene;

**L$^2$** is $C_{1-12}$alkylene; preferably $C_{1-6}$alkylene; more preferably $C_{1-2}$alkylene;

**L$^3$** is a single bond or $C_{1-12}$alkylene; wherein one carbon of the $C_{1-12}$alkylene is optionally replaced by one or more heteroatoms; preferably **L$^3$** is a single bond or $C_{1-12}$alkylene;

**Y** is hydrogen, halo, $C_{1-10}$alkyl, $C_{6-12}$aryl, $-OR^4$, $-COOR^4$, $-CONR^{5a}R^{5b}$, $-NR^{5a}R^{5b}$, $-SR^6$,$-SO_2R^7$, $-SiR^8{}_3$, $-OP(O)(OH)_2$, epoxy, or Het; each group being optionally substituted with one or more substituents each independently selected from halo, OH, $-OR^4$,$-COR^4$, $C_{1-6}$alkyl; $C_{6-12}$aryl, $C_{1-6}$alkoxy, oxo, halo$C_{1-6}$alkyl, and halo$C_{1-6}$alkoxy;

**Y$^1$** is $C_{6-12}$aryl substituted with one, two, or three substituents each independently selected from the group consisting of halo, halo$C_{1-6}$alkyl, and halo$C_{1-6}$alkoxy;

**Y$^2$** is $C_{6-12}$aryl substituted with one, two, or three substituents each independently selected from the group consisting of halo, halo$C_{1-6}$alkyl, and halo$C_{1-6}$alkoxy;

**Z** is a carboxylate ($-CO_2$), a sulfonate ($-SO_3$), a sulfinate ($-SO_2$), a phosphorus based anion (such as phosphonate, phosphite, phosphate and the like), an ester monosulfate ($-OSO_3$), or a sulfonamidate ($-NHSO_2$);

**A** is $[PF_6]$, $[AsF_6]$, $[SbF_6]$, $[N(SO_2CF_3)_2]$, $[BF_4]$, $[CF_3SO_3]$, $[CF_3CO_2]$, $[CH_3CO_2]$, $[CF_3SO_2]$, $[NO_2]$, $[NO_3]$, $[ClO_4]$, [Cl], [Br], [I], [HO-CO-O]; and $[AlR^{10}_{4-n}R^{11}_{n}]$;

$\mathbf{R^4}$ is hydrogen; or a group selected from $C_{1-6}$alkyl; $C_{6-12}$aryl; Het; and $C_{3-7}$cycloalkyl; each group being optionally substituted with one or more substituents each independently selected from $C_{1-6}$alkyl; $C_{3-7}$cycloalkyl, $C_{6-12}$aryl, halo, $C_{1-6}$alkoxy, oxo and Het;

$\mathbf{R^{5a}}$ and $\mathbf{R^{5b}}$ are, each independently, selected from hydrogen, $C_{1-6}$alkyl, and $C_{6-12}$aryl$C_{1-6}$alkyl;

$\mathbf{R^6}$ is hydrogen, $C_{1-6}$alkyl, $C_{6-12}$aryl, $C_{3-7}$cycloalkyl, or $-C(=O)R^9$;

$\mathbf{R^7}$ is hydrogen, $C_{1-6}$alkyl, or $C_{3-7}$cycloalkyl;

$\mathbf{R^8}$ is hydrogen, $C_{1-6}$alkyl optionally substituted with halo, $C_{1-6}$alkoxy, $C_{3-7}$cycloalkyl, or $C_{6-12}$aryl; $C_{3-7}$cycloalkyl; or $-OR^4$;

$\mathbf{R^9}$ is $C_{1-12}$alkyl;

$\mathbf{R^{10}}$ is Cl, Br, F or I or $C_{1-12}$alkyl;

$\mathbf{R^{11}}$ is F, Cl, Br, or I;

**n** is an integer from 0 to 4;

$\mathbf{C_{6-12}aryl}$ as a group or part of a group is phenyl, naphthyl, indanyl, or 1,2,3,4-tetrahydronaphthyl, each of which may be optionally substituted with one, two or three substituents selected from halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, mono- or di$C_{1-6}$alkylaminocarbonyl, azido, mercapto;

**Het** as a group or part of a group is a 5 or 6 membered saturated, partially unsaturated or completely unsaturated heterocyclic ring containing 1 to 4 heteroatoms each independently selected from nitrogen, oxygen and sulfur, said heterocyclic ring being optionally condensed with a benzene ring, and wherein the group Het as a whole may be optionally substituted with one, two or three substituents each independently selected from the group consisting of halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, and mono- or di$C_{1-6}$alkylaminocarbonyl.

It should be noted in all applicable embodiments that in the definition of $\mathbf{R^1}$, such substituent is hydrogen when $\mathbf{R^2}$, and $\mathbf{R^3}$, together with the atom **X,** i.e. nitrogen, form a non-aromatic ring and the nitrogen atom bears no double bond.

It should be further noted in all applicable embodiments that in the definition of $\mathbf{R^1}$, such substituent is not present when $\mathbf{R^2}$, and $\mathbf{R^3}$, together with the atom **X,** i.e. nitrogen, form an aromatic ring.

The only restriction being that the ionic liquid must not be eluted during the separation procedure.

The present invention also relates to a composition for use in chromatography comprising a substrate impregnated with an ionic liquid of formula (III) or (IV);

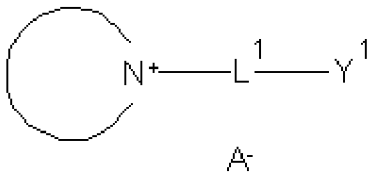

(III)

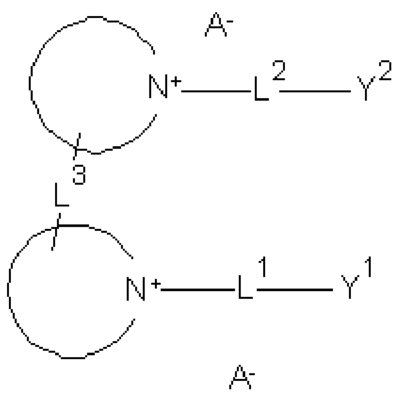

(IV)

wherein

each **ring** comprising the nitrogen atom is independently selected from the group consisting of pyridinium, pyrazinium, pyrrolium, imidazolium, pyrazolium, thiazolium, triazolium, indolium, tetrazolium, pyrimidinium, pyridazinium, piperazinium, and piperidinium; each group being optionally substituted with one, two, or three substituents selected from $C_{1-12}$alkyl and $C_{1-12}$alkoxy;

$\mathbf{L^1}$ is $C_{1-12}$alkylene; preferably $C_{1-6}$alkylene; more preferably $C_{1-2}$alkylene;

$\mathbf{L^2}$ is $C_{1-12}$alkylene; preferably $C_{1-6}$alkylene; more preferably $C_{1-2}$alkylene;

$\mathbf{L^3}$ is a single bond or $C_{1-12}$alkylene; wherein one carbon of the $C_{1-12}$alkylene is optionally replaced by one or more heteroatoms; preferably $\mathbf{L^3}$ is a single bond or $C_{1-12}$alkylene;

$\mathbf{Y^1}$ is $C_{6-12}$aryl substituted with one, two, or three substituents each independently selected from the group consisting of halo, halo$C_{1-6}$alkyl, and halo$C_{1-6}$alkoxy;

$\mathbf{Y^2}$ is $C_{6-12}$aryl substituted with one, two, or three substituents each independently selected from the group consisting of halo, halo$C_{1-6}$alkyl, and halo$C_{1-6}$alkoxy;

**A** is $[PF_6]$, $[AsF_6]$, $[SbF_6]$, $[N(SO_2CF_3)_2]$, $[BF_4]$, $[CF_3SO_3]$, $[CF_3CO_2]$, $[CH_3CO_2]$, $[CF_3SO_2]$, $[NO_2]$, $[NO_3]$, $[ClO_4]$, [Cl], [Br], [I], [HO-CO-O]; and $[AlR^{10}_{4-n}R^{11}_{n}]$;

$\mathbf{R^{10}}$ is Cl, Br, F or I or $C_{1-12}$alkyl;

$\mathbf{R^{11}}$ is F, Cl, Br, or I;

**n** is an integer from 0 to 4;

$\mathbf{C_{6-12}}$**aryl** as a group or part of a group is phenyl, naphthyl, indanyl, or 1,2,3,4-tetrahydronaphthyl, each of which may be optionally substituted with one, two or three substituents selected from halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, mono- or di$C_{1-6}$alkylaminocarbonyl, azido, mercapto;

Groups used in the definitions of the variables include all possible isomers unless otherwise indicated. For instance pyridyl includes 2-pyridyl, 3-pyridyl and 4-pyridyl; pentyl includes 1-pentyl, 2-pentyl and 3-pentyl.

Non-limiting example of suitable ionic liquids of formula (I) or (II) can be selected from ethylammonium nitrate, n-propylammonium nitrate, butylammonium thiocyanate, tetra-n-heptylammonium chloride, tetra-n-butylammonium 2-hydroxy-4-morpholinopropanesulfonate, 1-Butyl-3-ethylimidazolium bis(trifluoromethylsulfonyl)amide, 1-Butyl-3-ethylimidazolium perfluorobutanesulfonate, 1-Butyl-3-methylimidazolium Hexafluorophosphate, 1-Butyl-3-methylimidazolium tetrafluoroborate, 1-iso-Butyl-3-methylimidazolium bis(trifluoromethylsulfonyl)amide, 1-n-Decyl-3-methylyimidazolium tetrafluoroborate, 1,3-Diethylimidazolium trifluoromethanesulfonate, 1,3-Dimethylimidazolium bis(trifluoromethylsulfonyl)amide, 1-Ethyl-3-methylimidazolium trifluoroacetate, 1-n-Hexyl-3-methylimidazolium hexafluorophosphate, 1-n-Octyl-3-methylimidazolium tetrafluoroborate.

Further examples of suitable ionic liquids may be found in US5,994,602 or in WO96/18459 or in WO01/81353. They disclose various methods for preparing ionic liquids and various applications.

In a preferred embodiment, the stationary phase is impregnated with an ionic liquid of formula (I), (II), (III) or (IV) wherein

**X is N,** P, or Al;

**$R^1$, $R^2$, and $R^3$** are each independently a group selected from $C_{1-10}$alkyl or $C_{6-12}$aryl; each group being optionally substituted with one or more substituents each independently selected from halo, $C_{1-6}$alkyl, $C_{1-6}$alkoxy, cyano, polyhalo$C_{1-6}$alkoxy, $C_{3-7}$cycloalkyl, $C_{6-12}$aryl, oxo, and Het;

or **X** is N, **$R^1$** is hydrogen or is not present, and **$R^2$, and $R^3$**, together with the atom **X** to which they are attached to, form a ring selected from pyridinium, imidazolium, pyrazolium, thiazolium, triazolium, pyrrolium, indolium, tetrazolium, pyrimidinium, pyrazinium, pyridazinium, piperazinium, pyrrolidinium, morpholinium, and piperidinium;

each **ring** comprising the nitrogen atom is independently selected from the group consisting of pyridinium, pyrazinium, pyrrolium, imidazolium, pyrazolium, thiazolium, triazolium, indolium, tetrazolium, pyrimidinium, pyridazinium, piperazinium, and piperidinium; each group being optionally substituted with one, two, or three substituents selected from $C_{1-6}$alkyl and $C_{1-6}$alkoxy;

**L** is $C_{1-6}$alkylene, $C_{3-7}$cycloalkylene, $C_{6-12}$arylene; each of $C_{1-6}$alkylene, $C_{3-7}$cycloalkylene, and $C_{6-12}$arylene, optionally containing 1 to 4 heteroatoms each independently selected from nitrogen, oxygen and sulfur; and each of $C_{1-6}$alkylene, $C_{3-7}$cycloalkylene, and $C_{6-12}$arylene, being optionally substituted with one or more substituents each independently selected from $C_{1-6}$alkyl and oxo;

**$L^1$** is $C_{1-6}$alkylene;

**$L^2$** is $C_{1-6}$alkylene;

**$L^3$** is a single bond or $C_{1-6}$alkylene; wherein one carbon of the $C_{1-6}$alkylene is optionally replaced by one or more heteroatoms;

**Y** is hydrogen, halo, or a group selected from $C_{1-10}$alkyl, $C_{6-12}$aryl, $-OR^4$, $-COOR^4$, $-CONR^{5a}R^{5b}$, $-NR^{5a}R^{5b}$, $-SR^6$, $-SO_2R^7$, $-SiR^8_3$, $-OP(O)(OH)_2$, epoxy, or Het; each group being optionally substituted with one or more substituents each independently selected from halo, OH, $-OR^4$, $-COR^4$, $C_{1-6}$alkyl; $C_{6-12}$aryl, $C_{1-6}$alkoxy, and oxo;

**$Y^1$** is $C_{6-10}$aryl substituted with one, two, or three substituents each independently selected from the group consisting of halo, halo$C_{1-6}$alkyl, and halo$C_{1-6}$alkoxy;

**$Y^2$** is $C_{6-10}$aryl substituted with one, two, or three substituents each independently selected from the group consisting of halo, halo$C_{1-6}$alkyl, and halo$C_{1-6}$alkoxy;

**$Z^-$** is a carboxylate ($-CO_2^-$), a sulfonate ($-SO_3^-$), a sulfinate ($-SO_2^-$), a phosphorus based anion (such as phosphonate, phosphite, phosphate and the like), an ester monosulfate ($-OSO_3^-$), or a sulfonamidate ($-NHSO_2^-$);

**$A^-$** is $[Br^-]$, $[PF_6^-]$, $[AsF_6^-]$, $[SbF_6^-]$, $[N(SO_2CF_3)_2^-]$, $[BF_4^-]$, $[CF_3SO_3^-]$, $[CF_3CO_2^-]$, $[CH_3CO_2^-]$, $[CF_3SO_2^-]$, $[NO_2^-]$, $[NO_3^-]$, $[ClO_4^-]$, $[Cl^-]$, $[I^-]$, [HO-CO-O-]; or $[AlR^{10}_{4-n}R^{11}_n]$;

**$R^4$** is hydrogen; or a group selected from $C_{1-6}$alkyl; $C_{6-12}$aryl; Het; and $C_{3-7}$cycloalkyl; each group being optionally substituted with one or more substituents each independently selected from $C_{1-6}$alkyl; $C_{3-7}$cycloalkyl, $C_{6-12}$aryl, halo, $C_{1-6}$alkoxy, oxo, and Het;

**$R^{5a}$** and **$R^{5b}$** are, each independently, selected from hydrogen, $C_{1-6}$alkyl, and $C_{6-12}$aryl$C_{1-6}$alkyl;

**$R^6$** is hydrogen, $C_{1-6}$alkyl, $C_{6-12}$aryl, $C_{3-7}$cycloalkyl, or $-C(O)R^9$;

**$R^7$** is hydrogen, $C_{1-6}$alkyl or $C_{3-7}$cycloalkyl;

**$R^8$** is hydrogen, $C_{1-6}$alkyl optionally substituted with halo, $C_{1-6}$alkoxy, $C_{3-7}$cycloalkyl, or $C_{6-12}$aryl; $C_{3-7}$cycloalkyl; or $-OR^4$;

**$R^9$** is $C_{1-12}$alkyl;

**$R^{10}$** is Cl, Br, F, I, or $C_{1-12}$alkyl;

**$R^{11}$** is F, Cl, Br, or I;

**n** is an integer from 0 to 4;

**$C_{6-12}$aryl** as a group or part of a group is phenyl, naphthyl, indanyl, or 1,2,3,4-tetrahydronaphthyl, each of which may be optionally substituted with one, two or three substituents selected from halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, mono- or di$C_{1-6}$alkylaminocarbonyl, azido, and mercapto;

**Het** as a group or part of a group is a 5 or 6 membered saturated, partially unsaturated or completely unsaturated heterocyclic ring containing 1 to 4 heteroatoms each independently selected from nitrogen, oxygen and sulfur, said heterocyclic ring being optionally condensed with a benzene ring, and wherein the group Het as a whole may be optionally substituted with one, two or three substituents each independently selected from the group consisting of halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, and mono- or di$C_{1-6}$alkylaminocarbonyl.

In a preferred embodiment, the stationary phase is impregnated with an ionic liquid of formula (I), (II), or (III) or (IV), wherein

**X** is N, **$R^1$** is hydrogen or is not present, and **$R^2$**, and **$R^3$**, together with the atom **X** to which they are attached to, form a ring selected from pyridinium, imidazolium, pyrazolium, thiazolium, triazolium, pyrrolium, indolium, tetrazolium, pyrimidinium, pyrazinium, pyridazinium, piperazinium, and piperidinium; each optionally substituted with one, two, or three substituents selected from $C_{1-12}$alkyl and $C_{1-12}$alkoxy;

each **ring** comprising the nitrogen atom is independently selected from the group consisting of pyridinium, imidazolium, pyrazinium, pyrrolium, pyrazolium, thiazolium, triazolium, indolium, tetrazolium, pyrimidinium, pyridazinium, piperazinium, and piperidinium; each group being optionally substituted with one, two, or three substituents selected from $C_{1-12}$alkyl and $C_{1-12}$alkoxy, preferably each group being optionally substituted with one, two, or three substituents selected from $C_{1-6}$alkyl and $C_{1-6}$alkoxy;

**$L^1$** is $C_{1-6}$alkylene;

**$L^2$** is $C_{1-6}$alkylene;

**$L^3$** is a single bond, or $C_{1-6}$alkylene; wherein one carbon of the $C_{1-6}$alkylene is optionally replaced by one or two heteroatoms;

**$Y^1$** is phenyl substituted with one, two, or three substituents selected from the group consisting of halo, halo$C_{1-6}$alkyl, and halo$C_{1-6}$alkoxy;

**$Y^2$** is phenyl substituted with one, two, or three substituents selected from the group consisting of halo, halo$C_{1-6}$alkyl, and halo$C_{1-6}$alkoxy;

**L** is $C_{1-12}$alkylene; or $C_{6-12}$arylene; preferably $C_{1-6}$alkylene; more preferably $C_{1-2}$alkylene;

**Y** is hydrogen, halo, or a group selected from $C_{1-10}$alkyl, $C_{6-12}$aryl, $-OR^4$, $-COOR^4$, $-CONR^{5a}R^{5b}$, $-NR^{5a}R^{5b}$, $-SR^6$, $-SO_2R^7$, $-SiR^8_3$, $-OP(O)(OH)_2$, epoxy, and Het; each group being optionally substituted with one or more substituents

each independently selected from halo, OH, $-OR^4$, $-COR^4$, $C_{1-6}$alkyl; $C_{6-12}$aryl, $C_{1-6}$alkoxy, oxo, halo$C_{1-6}$alkyl, and halo$C_{1-6}$alkoxy; preferably **Y** is $C_{6-12}$aryl optionally substituted with one or more halo substituents such as chloro or bromo, halo$C_{1-6}$alkyl, and halo$C_{1-6}$alkoxy; more preferably **Y** is $C_{6-12}$aryl substituted with one, two, or three substituents each independently selected from the group consisting of halo, halo$C_{1-6}$alkyl, and halO$C_{1-6}$alkoxy;

**A** is [Br], [Cl], [$PF_6$], [$AsF_6$], [$SbF_6$], [$N(SO_2CF_3)_2$], [$BF_4$], [$CF_3SO_3$], [$CH_3CO_2$], [$CF_3SO_2$], [$NO_2$], [$NO_3$], [$ClO_4$], [I], or [$AlR^{10}{}_{4-n}R^{11}{}_n$]; preferably [Br] or [Cl];

$Z^-$ is $-CO_2$, $-SO_3$, $-SO_2$, $-PO_2$, $-PO_3$, $-OSO_3$, or $-NHSO_2$.

In a preferred embodiment, the stationary phase is impregnated with an ionic liquid of formula (III) or (IV), preferably (III), wherein

each **ring** comprising the nitrogen atom is independently selected from the group consisting of pyridinium, pyrazinium, pyrrolium, imidazolium, pyrazolium, thiazolium, triazolium, indolium, tetrazolium, pyrimidinium, pyridazinium, piperazinium, and piperidinium, each group being optionally substituted with one, two, or three substituents selected from $C_{1-12}$alkyl and $C_{1-12}$alkoxy; preferably each ring comprising the nitrogen atom is independently selected from the group consisting of pyridinium, pyrazinium, pyrrolium, imidazolium, pyrazolium, pyrimidinium, pyridazinium, piperazinium, and piperidinium, each group being optionally substituted with one, two, or three substituents selected from $C_{1-12}$alkyl and $C_{1-12}$alkoxy, preferably optionally substituted with one, two, or three substituents selected from $C_{1-6}$alkyl and $C_{1-6}$alkoxy; more preferably each ring comprising the nitrogen atom is independently selected from the group consisting of pyridinium, pyrazinium, pyrrolium, imidazolium, pyrazolium, pyrimidinium, and pyridazinium, each group being optionally substituted with one, two, or three substituents selected from $C_{1-12}$alkyl and $C_{1-12}$alkoxy, preferably optionally substituted with one, two, or three substituents selected from $C_{1-6}$alkyl and $C_{1-6}$alkoxy; even more preferably each ring comprising the nitrogen atom is independently selected from the group consisting of pyridinium, pyrazinium, pyrrolium, imidazolium, and pyrazolium, each group being optionally substituted with one, two, or three substituents selected from $C_{1-12}$alkyl and $C_{1-12}$alkoxy, preferably optionally substituted with one, two, or three substituents selected from $C_1$-$_6$alkyl and $C_{1-6}$alkoxy; also preferably each ring comprising the nitrogen atom is independently selected from the group consisting of pyridinium, pyrazinium, pyrrolium, and imidazolium, each group being optionally substituted with one, two, or three substituents selected from $C_{1-12}$alkyl and $C_{1-12}$alkoxy, preferably optionally substituted with one, two, or three substituents selected from $C_{1-6}$alkyl and $C_{1-6}$alkoxy; also preferably each ring comprising the nitrogen atom is independently selected from the group consisting of pyridinium, imidazolium, pyrazinium, and pyrrolium, each group being optionally substituted with one, two, or three substituents selected from $C_{1-12}$alkyl and $C_{1-12}$alkoxy, preferably optionally substituted with one, two, or three substituents selected from $C_{1-6}$alkyl and $C_{1-6}$alkoxy; most preferably each ring comprising the nitrogen atom is pyridinium, or imidazolium, , each being optionally substituted with one, two, or three substituents selected from $C_{1-12}$alkyl and $C_{1-12}$alkoxy, preferably optionally substituted with one, two, or three substituents selected from $C_{1-6}$alkyl and $C_{1-6}$alkoxy; preferably optionally substituted with one, two, or three $C_{1-6}$alkyl substituents;

$\mathbf{L^1}$ is $C_{1-12}$alkylene; preferably $\mathbf{L^1}$ is $C_{1-6}$alkylene; more preferably $\mathbf{L^1}$ is $C_{1-3}$alkylene; also preferably $\mathbf{L^1}$ is $C_{1-2}$alkylene;

$\mathbf{L^2}$ is $C_{1-12}$alkylene; preferably $\mathbf{L^2}$ is $C_{1-6}$alkylene; more preferably $\mathbf{L^2}$ is $C_{1-3}$alkylene; also preferably $\mathbf{L^2}$ is $C_{1-2}$alkylene;

$\mathbf{L^3}$ is a single bond or $C_{1-12}$alkylene; wherein one carbon of the $C_{1-12}$alkylene is optionally replaced by one or more heteroatoms; preferably $\mathbf{L^3}$ is a single bond or $C_{1-6}$alkylene; wherein one carbon of the $C_{1-6}$alkylene is optionally replaced by one or two heteroatoms selected from O, N or S; preferably $\mathbf{L^3}$ is a single bond or $C_{1-3}$alkylene; wherein one carbon of the $C_{1-3}$alkylene is optionally replaced by one heteroatoms selected from O, N or S; preferably $\mathbf{L^3}$ is a single bond or $C_{1-2}$alkylene;

$\mathbf{Y^1}$ is $C_{6-12}$aryl substituted with one, two, or three substituents each independently selected from the group consisting of halo, halo$C_{1-6}$alkyl, and halo$C_{1-6}$alkoxy; preferably $\mathbf{Y^1}$ is $C_{6-10}$aryl substituted with one, two, or three substituents each independently selected from the group consisting of halo, halo$C_{1-6}$alkyl, and halo$C_{1-6}$alkoxy; preferably $\mathbf{Y^1}$ is phenyl substituted with one, two, or three substituents each independently selected from the group consisting of halo, halo$C_{1-6}$alkyl, and halo$C_{1-6}$alkoxy; preferably $\mathbf{Y^1}$ is phenyl substituted with one, two, or three halo substituents; more preferably $\mathbf{Y^1}$ is phenyl substituted with one, two, or three substituents independently selected from fluoro, chloro or bromo; more preferably $\mathbf{Y^1}$ is phenyl substituted with one, two, or three substituents independently selected

from fluoro, or chloro; more preferably **$Y^1$** is phenyl substituted with one, two, or three chloro substituents;

**$Y^2$** is $C_{6-12}$aryl substituted with one, two, or three substituents each independently selected from the group consisting of halo, halo$C_{1-6}$alkyl, and halo$C_{1-6}$alkoxy; preferably **$Y^2$** is $C_{6-10}$aryl substituted with one, two, or three substituents each independently selected from the group consisting of halo, halo$C_{1-6}$alkyl, and halo$C_{1-6}$alkoxy; preferably **$Y^2$** is phenyl substituted with one, two, or three substituents each independently selected from the group consisting of halo, halo$C_{1-6}$alkyl, and halo$C_{1-6}$alkoxy; preferably **$Y^2$** is phenyl substituted with one, two, or three halo substituents; more preferably **$Y^2$** is phenyl substituted with one, two, or three substituents independently selected from fluoro, chloro or bromo; more preferably **$Y^2$** is phenyl substituted with one, two, or three substituents independently selected from fluoro, or chloro; more preferably **$Y^2$** is phenyl substituted with one, two, or three chloro substituents;

**A** is selected from the group consisting of [Br], [Cl], [$PF_6$], [$AsF_6$], [$SbF_6$], [$N(SO_2CF_3)_2$], [$BF_4$], [$CF_3SO_3$], [$CH_3CO_2$], [$CF_3SO_2$], [$NO_2$], [$NO_3$], [$ClO_4$], and [I], preferably **A** is selected from the group consisting of [Br], [Cl], [$PF_6$], [$AsF_6$], [$SbF_6$], [$BF_4$], [$CF_3SO_3$], [$CH_3CO_2$], [$CF_3SO_2$], and [I]; preferably **A** is selected from the group consisting of [Br], [$PF_6$], [$BF_4$], [Cl], [$CF_3SO_3$], and [I]; preferably **A** is selected from the group consisting of [Br], [$PF_6$], [$BF_4$].

Preferably, the ionic liquid is a compound of formula (III) or (IV) as depicted above, preferably of formula (III), wherein

each **ring** comprising the nitrogen atom is independently selected from the group consisting of pyridinium, imidazolium, pyrazinium, pyrrolium, pyrazolium, thiazolium, triazolium, indolium, tetrazolium, pyrimidinium, pyridazinium, piperazinium, and piperidinium; each group being optionally substituted with one, two, or three substituents selected from $C_{1-12}$alkyl and $C_{1-12}$alkoxy, preferably each group being optionally substituted with one, two, or three substituents selected from $C_{1-6}$alkyl and $C_{1-6}$alkoxy;

**$L^1$** is $C_{1-6}$alkylene;

**$L^2$** is $C_{1-6}$alkylene;

**$L^3$** is a single bond, or $C_{1-6}$alkylene; wherein one carbon of the $C_{1-6}$alkylene is optionally replaced by one or two heteroatoms;

**$Y^1$** is phenyl substituted with one, two, or three substituents selected from the group consisting of halo, halo$C_{1-6}$alkyl, and halo$C_{1-6}$alkoxy;

$Y^2$ is phenyl substituted with one, two, or three substituents selected from the group consisting of halo, halo$C_{1-6}$alkyl, and halo$C_{1-6}$alkoxy;

**A** is selected from the group consisting of [Br], [Cl], [$PF_6$], [$AsF_6$], [$SbF_6$], [$N(SO_2CF_3)_2$], [$BF_4$], [$CF_3SO_3$], [$CH_3CO_2$], [$CF_3SO_2$], [$NO_2$], [$NO_3$], [$ClO_4$], and [I].

Preferably, the ionic liquid used for impregnating the substrate is a compound of formula (III) or (IV), preferably (III), wherein

each **ring** comprising the nitrogen atom is independently selected from pyridinium, imidazolium; pyrazinium, and pyrrolium, each being optionally substituted with one, two, or three substituents selected from $C_{1-6}$alkyl and $C_{1-6}$alkoxy, preferably each optionally substituted with one, two, or three substituents selected from $C_{1-4}$alkyl and $C_{1-4}$alkoxy;

**$L^1$** is $C_{1-2}$alkylene;

**$L^2$** is $C_{1-2}$alkylene;

**$L^3$** is a single bond, or $C_{1-2}$alkylene;

**$Y^1$** is phenyl substituted with one, two, or three substituents selected from the group consisting of halo, halo$C_{1-6}$alkyl, and halo$C_{1-6}$alkoxy; preferably halo;

**$Y^2$** is phenyl substituted with one, two, or three substituents selected from the group consisting of halo, halo$C_{1-6}$alkyl, and halO$C_{1-6}$alkoxy; preferably halo;

**A** is selected from the group consisting of [Br], [Cl], [$PF_6$], [$AsF_6$], [$SbF_6$], [$N(SO_2CF_3)_2$], [$BF_4$], [$CF_3SO_3$], [$CH_3CO_2$], [$CF_3SO_2$], [$NO_2$], [$NO_3$], [$ClO_4$], and [I]; preferably **A** is selected from the group consisting of [Br], [$PF_6$], [$BF_4$], [Cl], [$N(SO_2CF_3)_2$], [$CF_3SO_3$], [$CH_3CO_2$], [$CF_3SO_2$], [$NO_2$], [$NO_3$] [$ClO_4$], and [I].

Preferably, the ionic liquid used is a compound of formula (Ia), or (Ib),

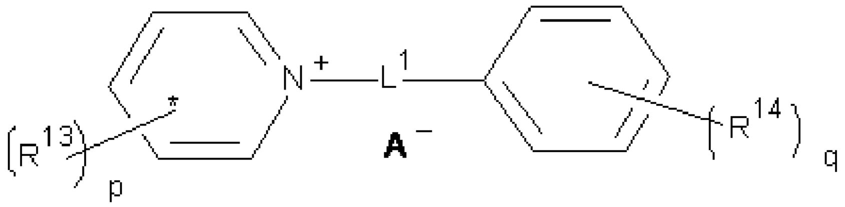

(Ia)

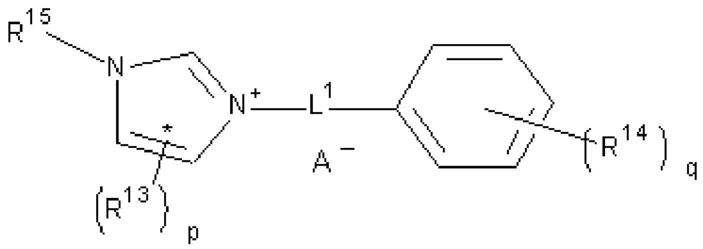

(Ib)

wherein

**A** is selected from the group consisting of [Br], [Cl], [$PF_6$], [$AsF_6$], [$SbF_6$], [$N(SO_2CF_3)_2$], [$BF_4$], [$CF_3SO_3$], [$CH_3CO_2$], [$CF_3SO_2$], [$NO_2$], [$NO_3$], [$ClO_4$], [I], and [$AlR^{10}_{4-n}R^{11}_n$]; and

**$R^{10}$** is halo or $C_{1-12}$alkyl; preferably halo or $C_{1-6}$alkyl;

**$R^{11}$** is halo; and

**n** is an integer selected from 0, 1, 2, 3, or 4;

**$L^1$** is $C_{1-6}$alkylene; preferably **$L^1$** is $C_{1-4}$alkylene; preferably **$L^1$** is $C_{1-2}$alkylene; preferably - $CH_2$-;

each **$R^{13}$** is independently selected from hydrogen, $C_{1-6}$alkyl and $C_{1-6}$alkoxy;

each **$R^{14}$** is independently selected from the group consisting of halo, halo$C_{1-6}$alkyl, and halo$C_{1-6}$alkoxy;

**$R^{15}$** is independently selected from $C_{1-6}$alkyl or hydrogen; preferably $C_{1-6}$alkyl;

**p** is an integer selected from 1, 2, or 3; and

**q** is an integer selected from 1, 2, or 3.

In a further embodiment, the ionic liquid is a compound of formula (Ia), or (Ib), as depicted above, wherein

**A** is selected from the group consisting of [Br], [Cl], [$PF_6$], [$AsF_6$], [$SbF_6$], [$BF_4$], [$CH_3CO_2$], [$CF_3SO_2$], [$NO_2$], [$NO_3$], [$ClO_4$], [I]; and

**$L^1$** is $C_{1-6}$alkylene; preferably **$L^1$** is $C_{1-4}$alkylene; more preferably **$L^1$** is $C_{1-2}$alkylene; preferably -$CH_2$-;

each **$R^{13}$** is independently selected from hydrogen, $C_{1-6}$alkyl and $C_{1-6}$alkoxy;

each **$R^{14}$** is independently selected from the group consisting of halo, halo$C_{1-6}$alkyl, and halo$C_{1-6}$alkoxy;

**$R^{15}$** is independently selected from $C_{1-6}$alkyl or hydrogen; preferably $C_{1-6}$alkyl;

**p** is an integer selected from 1, 2, or 3; and

**q** is an integer selected from 1, 2, or 3.

In a further embodiment, the ionic liquid is a compound of formula (Ia), or (Ib), as depicted above, wherein

**A** is selected from the group consisting of [Br], [Cl], [$PF_6$], [$AsF_6$], [$SbF_6$], [$BF_4$], [$CH_3CO_2$], [$CF_3SO_2$], [$NO_2$], [$NO_3$], [$ClO_4$], [I];

**$L^1$** is $C_{1-4}$alkylene; preferably $C_{1-2}$alkylene; preferably -$CH_2$-;

each **$R^{13}$** is independently selected from hydrogen, $C_{1-4}$alkyl and $C_{1-4}$alkoxy; preferably hydrogen

each **$R^{14}$** is independently selected from chloro, fluoro, and bromo, preferably chloro or fluoro ,

**$R^{15}$** is independently selected from $C_{1-4}$alkyl or hydrogen;

**p** is 1, 2 or 3, and

**q** is 1, 2, or 3.

In a further embodiment, the ionic liquid is a compound of formula (Ia), or (Ib), as depicted above, wherein

**A** is selected from the group consisting of [Br], [Cl], [$PF_6$], [$BF_4$], [$CH_3CO_2$], [$CF_3SO_2$], [I]; and

**$L^1$** is $C_{1-4}$alkylene; more preferably **$L^1$** is $C_{1-2}$alkylene; preferably -$CH_2$-;

each **$R^{13}$** is independently selected from hydrogen, $C_{1-6}$alkyl and $C_{1-6}$alkoxy;

each **$R^{14}$** is independently selected from the group consisting of halo, halo$C_{1-6}$alkyl, and halo$C_{1-6}$alkoxy;

**$R^{15}$** is independently selected from $C_{1-6}$alkyl or hydrogen; preferably $C_{1-6}$alkyl;

**p** is an integer selected from 1, 2, or 3; and

**q** is an integer selected from 1, 2, or 3.

In a further embodiment, the ionic liquid is a compound of formula (Ia), or (Ib), as depicted above, wherein

**A** is selected from the group consisting of [Br], [Cl], [$PF_6$], and [$BF_4$]; and

**$L^1$** is $C_{1-4}$alkylene; more preferably **$L^1$** is $C_{1-2}$alkylene; preferably -$CH_2$-;

each **$R^{13}$** is independently selected from hydrogen, $C_{1-6}$alkyl and $C_{1-6}$alkoxy;

each **$R^{14}$** is independently selected from the group consisting of halo, halo$C_{1-6}$alkyl, and halo$C_{1-6}$alkoxy;

**$R^{15}$** is independently selected from $C_{1-6}$alkyl or hydrogen; preferably $C_{1-6}$alkyl;

**p** is an integer selected from 1, 2, or 3; and

**q** is an integer selected from 1, 2, or 3.

In a further embodiment, the ionic liquid is a compound of formula (Ia), or (Ib), as depicted above, wherein

**A** is [Br], [$PF_6$], [$BF_4$];

**L$^1$** is $C_{1-2}$alkylene; preferably $-CH_2-$;

each **R$^{13}$** is independently selected from hydrogen, $C_{1-4}$alkyl and $C_{1-4}$alkoxy; preferably hydrogen

each **R$^{14}$** is independently selected from chloro, fluoro, and bromo, preferably chloro or, bromo,

**R$^{15}$** is independently selected from $C_{1-4}$alkyl or hydrogen; preferably $C_{1-2}$alkyl;

**p** is 1, 2 or 3, and

**q** is 1, 2, or 3.

In a further embodiment, the ionic liquid is a compound of formula (Ia), or (Ib) as depicted above, wherein

**A** is Br], [$PF_6$], or [$BF_4$],

**L$^1$** is $C_{1-2}$alkylene; preferably $-CH_2-$;

each **R$^{13}$** is hydrogen, or $C_{1-4}$alkyl;

each **R$^{14}$** is independently selected from chloro or bromo, and

**R$^{15}$** is independently selected from $C_{1-2}$alkyl or hydrogen;

**p** is 1, 2 or 3, and

**q** is 1, 2, or 3.

In a further embodiment, the ionic liquid is a compound of formula (Ia), or (Ib) as depicted above, wherein

**A** is [Br], [$PF_6$], [$BF_4$];,

**L$^1$** is $C_{1-2}$alkylene; preferably $-CH_2-$;

each **R$^{13}$** is hydrogen, or $C_{1-4}$alkyl; preferably hydrogen

each **R$^{14}$** is independently selected from chloro, and bromo,

**R$^{15}$** is independently selected from $C_{1-2}$alkyl or hydrogen ; preferably $C_{1-2}$alkyl;

**p** is 1, or 2, and

**q** is 1, 2, or 3.

In a further embodiment, the ionic liquid is a compound of formula (Ia), or (Ib) as depicted above, wherein

**A** is [Br], [$PF_6$], [$BF_4$];,

**L$^1$** is $C_{1-2}$alkylene; preferably $-CH_2-$;

each **R$^{13}$** is hydrogen,

each **R$^{14}$** is independently selected from chloro, and bromo,

**$R^{15}$** is independently selected from $C_{1-2}$alkyl or hydrogen ; preferably $C_{1-2}$alkyl;

**p** is 1, and

**q** is 2, or 3.

Preferably, the ionic liquid used is a compound as listed in Table 1.

Table 1

| Ionic liquid | structure |
|---|---|
| 1 | 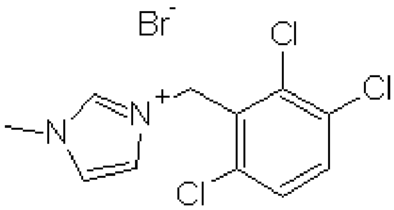 |
| 2 | 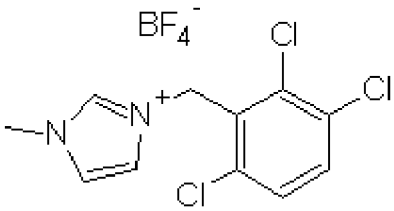 |
| 3 | 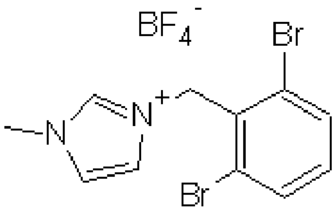 |
| 4 | 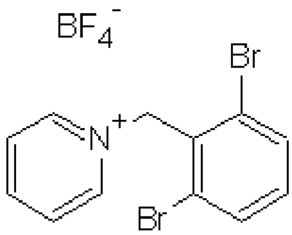 |
| 5 | 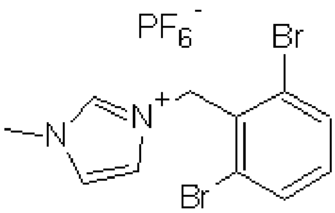 |
| 6 | 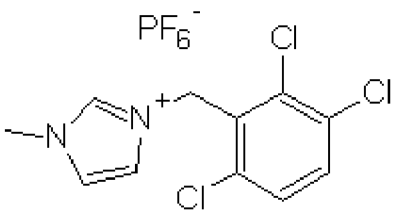 |

(continued)

| Ionic liquid | structure |
|---|---|
| 7 | 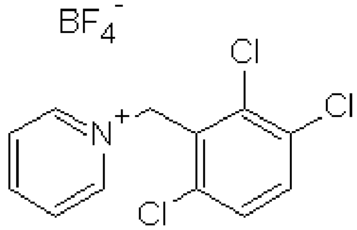 |
| 8 | 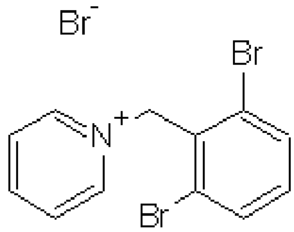 |
| 9 | 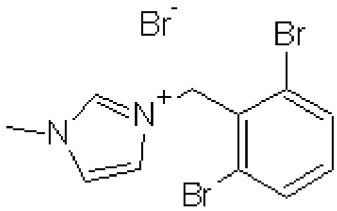 |
| 10 | 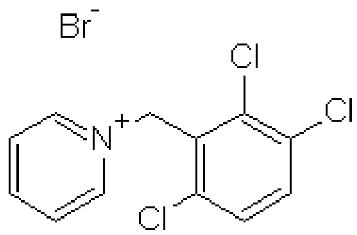 |

The ionic liquid compound of formula (III) or (IV) can be prepared by contacting a compound of formula (Ig) with a compound of formula (Ih) thereby forming the compound of formula (III)

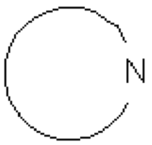

(Ig)

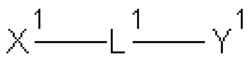

(Ih)

wherein $X^1$ is halo, and the nitrogen-containing ring is selected from the group consisting of pyridine, imidazole, pyrazine, pyrrole, pyrazole, thiazole, triazole, indole, tetrazole, pyrimidine, pyridazine, piperazine, and piperidine; each group being optionally substituted with one, two, or three substituents selected from $C_{1-12}$alkyl and $C_{1-12}$alkoxy; and $L^1$, and $Y^1$ are as defined in any one of the embodiments presented herein.

The contacting step can be done at room temperature in a suitable solvent such as ethyl acetate.

Once compound of formula (III) is prepared, different counter ion A, can be substituted with each other, by contacting the compound of formula (III) with the desired salt of said counter ion.

The impregnation of the planar stationary phase with an ionic liquid of formula (I), (II), (III), (IV); (Ia), or (Ib), may be performed by simply pouring or spraying the ionic liquid pure or in solution onto the planar stationary phase, and optionally allowing the stationary phase to dry. Alternatively, the impregnation may be performed by covalent anchoring of non-coordinating anions on mineral supports to prepare supported ionic liquids as described in US2011/0178258.

In one embodiment of the present invention, the substrate comprises silica based normal and reverse-phase resin optionally derivatized with alkyl groups or aromatic groups, preferably silica gel 60F254.

One embodiment of the present invention relates to the use of a composition according to the present invention as a stationary phase in chromatography.

One embodiment of the present invention relates to a method of performing planar chromatography characterized in that the planar stationary phase is a composition according to the present invention.

One embodiment of the present invention relates to a method of performing planar chromatography characterized in that the planar stationary phase is a composition according to the present invention, in a method for analyzing, separating, or characterizing chemical compounds, preferably polymers selected from polyolefins, polyamides, polycarbonates, poly(hydroxy carboxylic acid) like polylactic acid, polystyrenes, polyesters polyethylene terephthalate (PET), polybutylene terephthalate (PBT), polymethylmethacrylate (PMMA), poly(methyl acrylate) (PMA), vinyl polymers, proteins, and polysaccharides; or blends thereof.

In a preferred embodiment, the present invention also relates to a method of performing planar chromatography characterized in that the planar stationary phase is impregnated with an ionic liquid of formula (I), (II), (III), (IV), (Ia), or (Ib), wherein $\mathbf{R^1}$, $\mathbf{R^2}$, $\mathbf{R^3}$, **X, L,** $\mathbf{L^1}$, $\mathbf{L^2}$, $\mathbf{L^3}$, **Y,** $\mathbf{Y^1}$, $\mathbf{Y^2}$, **Z, A,** $\mathbf{R^{13}}$, $\mathbf{R^{14}}$, $\mathbf{R^{15}}$, **p, q,** and each **ring** have the same meaning as that defined herein above.

In a preferred embodiment, the present invention also relates to a method of performing planar chromatography characterized in that the planar stationary phase is impregnated with an ionic liquid of formula (I), (II), (III), (IV), (Ia), or (Ib), in a method for analyzing, separating, or characterizing chemical compounds, preferably polymers selected from polyolefins, polyamides, polycarbonate, poly(hydroxy carboxylic acid) like polylactic acid, polystyrenes, polyesters polyethylene terephthalate (PET), polybutylene terephthalate (PBT), polymethylmethacrylate (PMMA), poly(methyl acrylate) (PMA), vinyl polymers, proteins, and polysaccharides; or blends thereof.

The polymers to be analyzed, separated and/or characterized in the present invention can be produced by any method known in the art. Their production therefore is well known to the person skilled in the art and need not be described further. Preferably, the polymers are selected from the group comprising polyolefins, polyamides, polycarbonates, poly(hydroxy carboxylic acid), polystyrenes, polyesters, polyethylene terephthalate (PET), polybutylene terephthalate (PBT), polymethylmethacrylate (PMMA), poly(methyl acrylate) (PMA), vinyl polymers, proteins, and polysaccharides, or blends thereof.

The chemical compounds, preferably the polymers, are present in a sample. The sample can be of natural or synthetic origin. It can be provided as a liquid sample, where the analytes are typically present in a solvent or mixtures of solvents. The sample can comprise any desired further solid, emulsified or dissolved constituents, which, however, should interfere neither with the planar separation nor with the later optional staining of the analytes for visualization purposes.

Solid samples are generally firstly taken up in one of the below-mentioned solvents in order that they can be applied to the chromatography plate. In the case of concentrated samples, it may be necessary firstly to dilute them. It is known to the person skilled in the art in the area of planar chromatography how large the amount of sample and sample concentration may or must be, depending on the type of plate employed and the particular separation problem, in order to obtain bands which can be evaluated as ideally as possible.

The polymers to be analyzed, separated and/or characterized in the present invention can be produced by any method known in the art. Their production therefore is well known to the person skilled in the art and need not be described further. Preferably, the polymers are selected from the group comprising polyolefins, polyamides, polycarbonates, poly(hydroxy carboxylic acid), polystyrenes, polyesters, polyethylene terephthalate (PET), polybutylene terephthalate (PBT), polymethylmethacrylate (PMMA), poly(methyl acrylate) (PMA), vinyl polymers, proteins, and polysaccharides, or blends thereof.

In a preferred embodiment, the method is particularly useful for the separation and/or characterization of polyolefins. The polyolefins to be analyzed, separated, and/or characterized in the present invention may be any olefin homopolymer or any copolymer of an olefin and one or more comonomers. The polyolefins may be atactic, syndiotactic or isotactic. The olefin can for example be ethylene, propylene, 1-butene, 1-pentene, 1-hexene, 4-methyl-1-pentene or 1-octene, but also cycloolefins such as for example cyclopentene, cyclohexene, cyclooctene or norbornene. The comonomer may be different from the olefin and chosen such that it is suited for copolymerization with the olefin. The comonomer may be an olefin as defined above. Further examples of suitable comonomers are vinyl acetate ($H_3C\text{-}C(=O)O\text{-}CH=CH_2$) or vinyl alcohol ("$HOCH=CH_2$"), acrylate, methacrylate or styrene. Examples of olefin copolymers that can be analyzed, separated, or characterized in the present invention are random copolymers of propylene and ethylene, random copolymers of propylene and 1-butene, heterophasic copolymers of propylene and ethylene, ethylene-butene copolymers, ethylene-hexene copolymers, ethylene-octene copolymers, copolymers of ethylene and vinyl acetate (EVA), copolymers of ethylene and vinyl alcohol (EVOH).

Most preferred polyolefins to be analyzed, separated, or characterized in the present invention are olefin homopolymers and copolymers of an olefin and optionally one or more comonomers, wherein said olefin and said one or more comonomer are different. Preferably, said olefin is ethylene or propylene. The term "comonomer" refers to olefin comonomers which are suitable for being polymerized with olefin monomers, preferably ethylene or propylene monomers. Comonomers may comprise but are not limited to aliphatic $C_2$-$C_{20}$ alpha-olefins. Examples of suitable aliphatic $C_2$-$C_{20}$ alpha-olefins include ethylene, propylene, 1-butene, 4-methyl-1-pentene, 1-hexene, 1-octene, 1-decene, 1-dodecene, 1-tetradecene, 1-hexadecene, 1-octadecene and 1-eicosene. Preferred polyolefins for use in the present invention are propylene and ethylene polymers. As used herein, the terms "propylene polymer" and "polypropylene" as well as the terms "ethylene polymer" and "polyethylene" are used interchangeably. Preferably, the polyolefin is selected from polyethylene and polypropylene homo- and copolymers.

In some embodiments, the method is particularly useful for the separation and/or characterization of polyamides. Polyamides are characterized in that the polymer chain comprises amide groups (-NHC(=O)-). Polyamides to be analyzed, separated, or characterized in the present invention have preferably one of the following chemical structures: $[-NH-(CH_2)_n-C(=O)-]_x$, or $[-NH-(CH_2)_m-NH-C(=O)-(CH_2)_n-C(=O)-]_x$

wherein m and n may be independently chosen from one another and be an integer from 1 to 20.

Specific examples of suitable polyamides are polyamides 4, 6, 7, 8, 9, 10, 11, 12, 46, 66, 610, 612, and 613.

The polystyrenes to be analyzed, separated, or characterized in the present invention may be any styrene homopolymer or copolymer. They may be atactic, syndiotactic or isotactic. Styrene copolymers comprise one or more suitable comonomers, i.e. polymerizable compounds different from styrene. Examples of suitable comonomers are butadiene, acrylonitrile, acrylic acid or methacrylic acid and corresponding esters. Examples of styrene copolymers that may be analyzed, separated, or characterized in the present invention are butadiene-styrene copolymers, which are also referred to as high-impact polystyrene (HIPS), acrylonitrile-butadiene-styrene copolymers (ABS) or styreneacrylonitrile copolymers (SAN).

Polyesters that may be analyzed, separated, or characterized in the present invention are preferably having the following chemical structure $[-C(=O)-C_6H_4-C(=O)O-(CH_2-CH_2)_n-O-]_x$ wherein n is an integer from 1 to 10, with preferred values being 1 or 2.

Specific examples of suitable polyesters are polyethylene terephthalate (PET) and polybutylene terephthalate (PBT).

Furthermore, preferred polyesters are poly(hydroxy carboxylic acid)s as described below.

The poly(hydroxy carboxylic acid)s to be analyzed, separated, or characterized in the present invention can be any polymer wherein the monomers comprise at least one hydroxyl group and at least carboxyl group. The hydroxy carboxylic acid monomer is preferably obtained from renewable resources such as corn and rice or other sugar- or starch-producing plants. The term "poly(hydroxy carboxylic acid)" includes homo- and copolymers herein.

The poly(hydroxy carboxylic acid) can be represented as in Formula V:

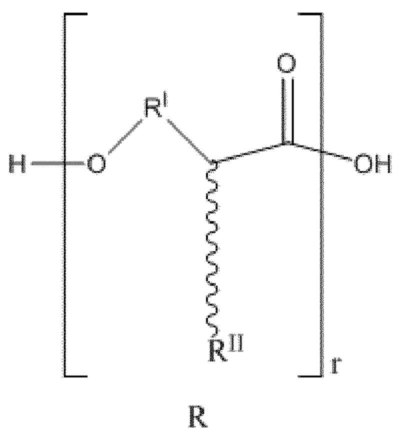

Formula V

wherein R" is hydrogen or a branched or linear alkyl comprising from 1 to 12 carbon atoms; $R^1$ is optional and can be a branched, cyclic or linear alkylene chains comprising from 1 to 12 carbon atoms; and "r" represents the number of repeating units of R and is any integer from 30 to 15000.

The monomeric repeating unit is not particularly limited, as long as it is aliphatic and has a hydroxyl residue and a carboxyl residue. Examples of possible monomers include lactic acid, glycolic acid, 3-hydroxybutyric acid, 4-hydroxybutyric acid, 4-hydroxyvaleric acid, 5-hydroxyvaleric acid, 6-hydroxycaproic acid and the like.

The monomeric repeating unit may also be derived from a cyclic monomer or cyclic dimer of the respective aliphatic hydroxycarboxylic acid. Examples of these include lactide, glycolide, β-propiolactone, β-butyrolactone, γ-butyrolactone, γ-valerolactone, 5-valerolactone, ε-caprolactone and the like.

In the case of asymmetric carbon atoms within the hydroxy carboxylic acid unit, each of the D-form and the L-form as well as mixtures of both may be present. Racemic mixtures can also be present. The term "poly(hydroxy carboxylic acid)" also includes blends of more than one poly(hydroxy carboxylic acid). The poly(hydroxy carboxylic acid) may optionally comprise one or more comonomers. The comonomer can be a second different hydroxycarboxylic acid as defined above in Formula V. The weight percentage of each hydroxycarboxylic acid is not particularly limited. The comonomer can also comprise dibasic carboxylic acids and dihydric alcohols. These react together to form aliphatic esters, oligoesters or polyesters having a free hydroxyl end group and a free carboxylic acid end group, capable of reacting with hydroxy carboxylic acids, such as lactic acid and polymers thereof. The poly(hydroxy carboxylic acid) can be preferably a polylactic acid (PLA). Preferably the polylactic acid is a homopolymer obtained either directly from lactic acid or from lactide, preferably from lactide.

One embodiment of the present invention relates to a method of performing planar chromatography characterized in that the planar stationary phase is impregnated with a compound of formula (I), (II), (III), (IV), (Ia), or (Ib), further characterized in that the chromatography run is performed at a working temperature between 10°C and 250°C, for example between 25°C and 100°C, preferably between 25°C and 50°C.

One embodiment of the present invention relates to a method of performing planar chromatography, characterized in that the planar stationary phase is a composition according to the present invention or in that the planar stationary phase is impregnated with a compound of formula (I), (II), (III), (IV), (Ia), or (Ib), further characterized in that the chromatography is performed at a working temperature between 30°C and 250°C, for example between 35°C and 200° C, preferably between 45°C and 200°C, more preferably between 50°C and 200°C, for example between 60°C and 200°C, for example between 70°C and 200°C, for example between 80°C and 200°C, preferably between 90°C and 200°C, preferably between 100°C and 200°C, preferably between 110°C and 200°C.

One embodiment of the present invention relates to a method of performing planar chromatography wherein the planar stationary phase is impregnated with a compound of formula (I), (II), (III), (IV), (Ia), or (Ib), and the chromatography is performed at a working temperature between 30°C and 250°C; for example between 35°C and 200°C, preferably between 45°C and 200°C, more preferably between 50°C and 200°C, for example between 60°C and 200°C, for example between 70°C and 200°C, for example between 80°C and 200°C, preferably between 90°C and 200°C, preferably between 100°C and 200°C, preferably between 110°C and 200°C, in a method for analyzing, separating, or characterizing chemical compounds, preferably polymers selected from polyolefins, polyamides, polycarbonates, poly(hydroxy carboxylic acid) like polylactic acid, polystyrenes, polyesters, polyethylene terephthalate (PET), polybutylene terephthalate (PBT), polymethylmethacrylate (PMMA), poly(methyl acrylate) (PMA), vinyl polymers, proteins, and polysaccharides; or blends thereof.

In one embodiment of the present invention, the chromatography is performed with a temperature gradient. A temperature gradient can be applied either to the stationary phase, or to the mobile phase, or the chromatography plate or to all using suitable device that is used to thermally treat in a controlled manner the planar chromatography. In one embodiment, the mobile phase and/or the stationary phase is subjected to a temperature gradient. The temperature gradient may be formed either as a temporal temperature gradient applied to the entire stationary phase or as a spatial temperature gradient in the stationary phase through which the sample migrate. In an embodiment, the temperature gradient is applied to the mobile phase. The temperature of the mobile phase can be controlled by an external heating system. The stationary phase can be controlled by internal heating plate in contact with the support on which the stationary phase is provided.

In an embodiment, the temperature gradient can be greater than, or equal to 1°C per minute, for example greater than, or equal to 2°C per minute, preferably greater than, or equal to 3°C per minute, preferably equal greater than, or equal to 5°C per minute, for example greater than, or equal to 8°C per minute, for example greater than, or equal to 10°C per minute.. Temperature can be ramped or increased step-wise or linearly. In an embodiment, the temperature is increased step-wise. For example, the chromatography can be started at a temperature T initial, then after x minutes, a temperature T1 is applied, then after y minutes a temperature T2 is applied. The mobile phase can be stopped during equilibration phase.

In one embodiment of the present invention, the chromatography can be performed under external pressure by applying either i) a neutral gas under pressure applied to a membrane on the planar stationary phase; ii) a pneumatic pillow or metallic membrane on the planar stationary phase; or iii) a pneumatic press on the planar stationary phase.

In one embodiment of the present invention, the stationary phase is subjected to a positive pressure differential of 2-3 bar relative to the pressure on the mobile phase, with a maximum pressure of 150 bar, by applying either i) a neutral gas under pressure applied to a membrane on the planar stationary phase; ii) a pneumatic pillow or metallic membrane on the planar stationary phase; or iii) a pneumatic press on the planar stationary phase.

The ranges for temperature of the stationary phase, flow rate of the eluent and pressure used in the process according to the invention depend on the particle size of the substrate material used in the separating plane, so that in an individual case the ranges given as well as the preferred ranges for these parameters can vary somewhat.

In one embodiment of the present invention, the chromatography is performed using a mobile phase is selected from the non-limiting group comprising toluene; xylene; tetrahydrofuran; $C_4$-$C_{20}$ branched or non-branched alkane such as heptane, hexane, or isobutane; supercritical carbon dioxide; trichlorobenzene; ethyl acetate; dimethylsulfoxide; dimethylformamide; a ionic liquid of formula (I), (II), (III), (IV), (Ia), or (Ib); and mixtures thereof; optionally in the presence of a co-eluent selected from the group comprising an alcohol and an ionic liquid of formula (I), (II), (III), (IV), (Ia), or (Ib), with the proviso that the ionic liquid of formula (I), (II), (III), (IV), (Ia), or (Ib), impregnating the stationary phase is not eluted with the mobile phase.

Examples of particularly suited alcohols to be used as co-eluents are methanol, ethanol, propanol, isopropanol, 1-butanol, 2-butanol, 2-methyl-1-propanol, 1-pentanol, 2-pentanol, 3-pentanol, 2-methyl-1-butanol, 2-methyl-2-butanol, 3-methyl-1-butanol and 3-methyl-2-butanol. Preferred alcohols are methanol, ethanol, propanol and isopropanol.

In one embodiment of the present invention, the chromatography is subjected to a gradient elution. In one embodiment of the present invention, the gradient elution comprises increasing the elution strength of the mobile phase between two subsequent chromatography steps. The stationary phase is allowed to dry between two subsequent chromatography steps. In one embodiment of the present invention, the method comprises at least two subsequent chromatography steps with progressively decreasing migration distances, wherein said steps are performed on the same planar chromatography device.

Preferably the method comprises loading one or more samples onto a planar stationary phase and while retaining said one or more samples on the planar stationary phase, subjecting said sample to planar chromatography (chromatographic separation) using a mobile phase; wherein in the chromatography is chromatography is performed with a gradient elution of the mobile phase and is repeated at least twice with progressively decreasing migration distances of the mobile phase.

Preferably, the chromatography is subjected to a gradient elution and comprises at least two subsequent chromatography steps with progressively decreasing migration distances of the mobile phase on the planar chromatography plate, wherein said gradient elution comprises increasing the elution strength of the mobile phase between two subsequent chromatography steps, said steps being performed on the same planar chromatography plate.

This type of multiple chromatography is a repeated procedure with different solvents or solvent mixtures eluting in the same direction with decreasing migration distances. A weaker eluent is used for the first chromatography, and the solvent front is allowed to migrate for about the total planned migration distance. This procedure is consecutively repeated each time with solvents or solvent mixtures of higher elution strength but the migration distances is reduced for each stage. This procedure is consecutively repeated and performed on the same planar chromatography device. Between each migration the same plate is suitably dried.

Using this technique, chromatographic separations are performed in the same direction with a stepwise elution gradient that becomes progressively stronger (increasing ST value) over distances that decrease by about 2 to 20 mm for each stage.

The planar chromatography can be performed on a plate coated with a layer of stationary phase, usually bound to an inert support. The stationary phase (also referred as active layer or sorbent bed) can be selected from a finely divided polar substance such as for example silica gel or aluminium oxide or cellulose.

Typically a solution of the sample to be characterized is deposited on the stationary phase as a small, tight spot and is then allowed to dry. The plate can then be inserted into a tank, a reactor or an overpressurized layer chromatography device provided. The chromatography (also referred herein as elution or migration) is carried out by capillary action generally in an ascending mode, or by forced flow through the stationary phase.

In one embodiment of the present invention, the planar chromatography is selected from a Thin-layer chromatography (TLC), High Performance Thin-Layer Chromatography (HPTLC), and Overpressurized Layer Chromatography (OPLC).

In one embodiment of the present invention, the planar stationary phase is selected from the group comprising silica based normal and reverse-phase thin layer chromatography resin optionally derivatized with alkyl groups or aromatic groups.

In one embodiment, the sorbent bed or plate is pre-treated by wetting with a mixture followed by drying. It creates a film on the surface of the support and it is selected in order to control and/or modify the separation conditions and revelation of the polymers under study. The mixture can be a combination of solvent and additives, it can be selected for example from toluene, xylene, tetrahydrofuran, $C_4$-$C_{20}$ branched or non-branched alkane such as heptane, hexane, or isobutane, supercritical carbon dioxide, trichlorobenzene, halogenated solvent, ethyl acetate, dimethylsulfoxide, dimethylformamide, an ionic liquid of formula (I), (II), (III), (IV), (Ia), or (Ib), inorganic salts, organic molecules, and mixtures thereof; the only restriction being that it must not be eluted during the separation procedure. It is selected in function of the polymer under study.

The solvent used to dissolve the polymer samples is selected in order to completely dissolve said polymer. If necessary, the solution is heated to achieve complete dissolution of the polymer. Complete dissolution of the polymers allows its homogeneous deposition on the sorbent bed. Typical concentrations range between 0.2 and 50 mg/mL,

preferably from 1 to 20 mg/mL, more preferably from 1 to 10 mg/mL, more preferably from 1 to 5 mg/mL. A higher concentration results in a better visualization of the migrated sample, but saturation must be avoided. It is adapted to the nature of the polymer and to the nature of the stationary phase.

Small amounts of each polymer solution are then deposited on the starting line of the sorbent bed, at position $R_F$=0. The amount of solution deposited ranges between 0.1 and 10 μL. Multiple depositions are also possible with drying between each deposition. It is allowed to dry before being submitted to elution.

In some embodiments, the polymers are studied by TLC or HPTLC. The sorbent bed is placed vertically in a tank containing a few millimeters of eluent and separation spontaneously occurs through ascending capillarity.

In another preferred embodiment, OPLC is used to characterize the polymers.

In an embodiment, the planar chromatography performed under pressure as described above, is performed using Overpressure Liquid Chromatography (OPLC). OPLC is based on the same principle as TLC, except that the ascending capillarity is replaced by a forced flow process through the stationary phase by applying pressure to the system. The commercially available OPLC system comprises a metal cassette supporting the sorbent bed. It is covered with sheet such as a Teflon sheet pierced with 2 parallel slits, one to let the mobile phase in and the other to let the mobile phase out. The system also comprises a seal around its entire perimeter. A pressure of is applied to the system in order to seal it. The mobile phase can then be introduced through the in-slit of the Teflon plate under a pressure, it flows through the system and is recovered at the out-slit of the Teflon plate.

In one embodiment of the present invention, when using OPLC, the mobile phase is injected at a pressure of 2 to 100 bar, preferably at a pressure of 5 to 80 bar, preferably at a pressure of 2 to 50 bar, preferably at a pressure of 2 to 20 bar, more preferably at a pressure of 2 to 10 bar.

In one embodiment of the present invention, the flow rate of the mobile phase is of 0.1 to 5 mL/minute. The mobile phase is preferably pumped through the stationary phase at a flow rate of 0.1 to 5 mL/minute, preferably at a flow rate of 0.2 to 3 mL/minute.

In one embodiment of the present invention, the planar stationary phase is subjected to a positive external pressure differential of 2-3 bar relative to the injection pressure on the mobile phase, with a maximum pressure of 150 bar.

OPLC allows the study of polymers having molecular weights ranging from dimers up to 1,500,000 Da.

In some embodiments, the stationary phase, loaded with polymer solutions is covered with a Teflon sheet and sealed by applying an external pressure corresponding to 4 to 103 bar, preferably of 5 to 80 bar, more preferably of 5 to 50 bar, more preferably of 5 to 30 bar, more preferably of 5 to 10 bar. The eluent can then be injected through the in-slit of the Teflon sheet at an inferior pressure of 2 to 3 bars relative to the pressure applied to the planar stationary phase, producing an eluent flow ranging between 0.1 and 5 mL/minute, preferably between 2 and 3 mL/minute.

The sorbent bed can have usually a size of from 10 to 20 cm and allows the simultaneous study of from 5 to 40 polymer samples in a period of time ranging between 2 and 30 minutes, preferably 2 to 5 minutes, more preferably about 3 minutes. It is therefore very advantageous as compared to GPC that requires a period of time of at least 15 minutes per sample. Preferably, a first migration is interrupted when the fastest sample has reached the end of the sorbent bed, at position $R_F$=1.

The choice of the eluent is determined by the nature of the polymer to be studied or by its expected molecular weight. It is possible to vary the eluent during the process in order to favor the migration of certain molecular weight ranges. For example, a first eluent can be selected to dissolve only the low molecular weight fraction in a polymer and thus migrate it. The eluent can then be modified to dissolve the next molecular weight fraction, and so on, until complete characterization of the polymer is obtained. Alternatively, a mixture of eluents with variable ratio of components can be used. For example, a mixture of heptane and ethyl acetate (AcOEt) with a ratio heptane/AcOEt ranging between 70/30 and 50/50 can be used to migrate the low to high molecular weight fractions.

The visualization of the results is carried out either directly if the polymers are colored, or with UV light or with a scanner, or with prior chemical or physical treatment of the plate.

In one embodiment, the methods of the present invention may be applied in a high-throughput screening.

The analytical plates may be any commercially available TLC plate, preferably with dimensions 200 x 200 x 0.2 mm. These analytical plates are preferably made of aluminum foil onto which a 60 Å silica gel is deposited. These types of plates guarantee a uniform and smooth surface and are physically resistant to cracks and deformations. They are impregnated with the ionic liquids of formula (I), (II), (III), (IV), (Ia), or (Ib), as described herein. They can also be impregnated with various compounds, organic or inorganic, such as fluorescent indicators for UV detection or other compounds facilitating the molecular separation. The upper surface of the plate is preferably surrounded by a fixed flexible seal of about 2 mm thickness. This seal crosses the entire thickness of the silica gel substrate and the impregnating layer of ionic liquid of formula (I), (II), (III), (IV), (Ia), or (Ib), and provides an hermetic analytical plate vis-a-vis the eluent. The analytical plate is preferably insertable and recoverable in a simple manner, taking care of the upper surface onto which the samples are arranged.

Further combinations or preferred embodiments are disclosed in the claims.

The present invention can be further illustrated by the following examples, although it will be understood that

these examples are included merely for purposes of illustration and are not intended to limit the scope of the invention unless otherwise specifically indicated.

**Examples**

The ionic liquids suitable for use in impregnation have been prepared as described below: The structures of the ionic liquids is given in Table 2.

Table 2

| Ionic liquid | structure | Melting point |
|---|---|---|
| 1 | 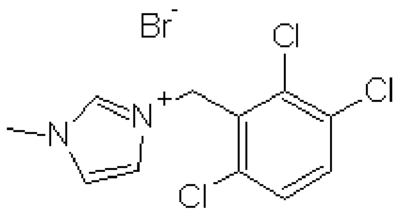 | 118°C |
| 2 | 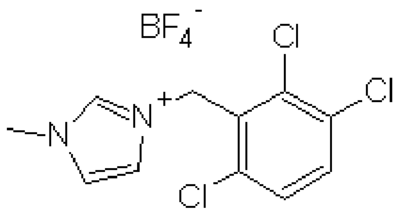 | 126°C |
| 3 | 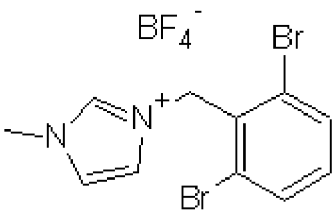 | 139°C |
| 4 | 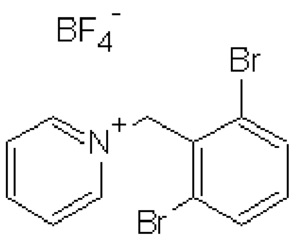 | 164°C |
| 5 | 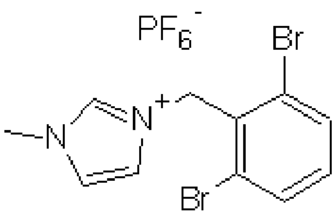 | 164°C |
| 6 | 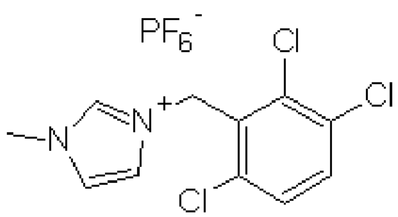 | 165°C |

(continued)

| Ionic liquid | structure | Melting point |
|---|---|---|
| 7 | 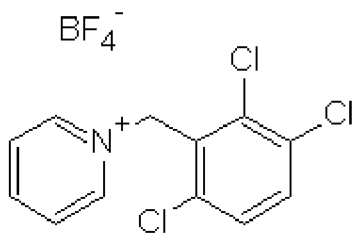 | 180°C |
| 8 | 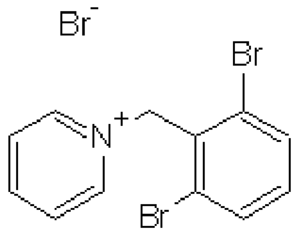 | 223°C |
| 9 | 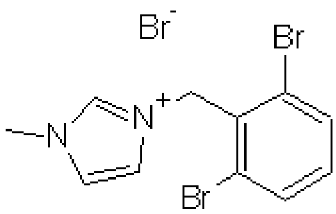 | 224°C |
| 10 | 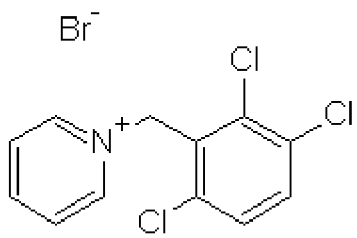 | 231°C |

**Ionic liquids 1:**

242μl of N-methyl-imidazole have been added to a round bottom flask. 1g of 2,3,6-trichlorobenzylbromide dissolved in 3mL of ethyl acetate was subsequently added to the round bottom flask at room temperature. After few minutes, a precipitate was obtained which was filtrated and washed several times with ethyl acetate. 594 mg (yield:55%) of a white solid was obtained having a melting point of 117.85°C.

**Ionic liquid 2:**

Two solutions were prepared:

1) 200mg of ionic liquid 1 was dissolved in 3ml of distilled water.

2) 74 mg of sodium tetrafluoroborate was dissolved in 2 ml of distilled water.

The two solutions were mixed and a white precipitate was immediately formed. The aqueous solution containing the precipitate was extracted several times with dichloromethane. The organic phases were combined, washed several time with water and subsequently dried using magnesium sulfate. The dichloromethane was removed under vacuum and 94 mg (yield:45%) of a white solid was obtained having a melting point of 139.48°C.

**Ionic liquid 3:**

Two solutions were prepared:

1) 3.22g of ionic liquid 9 was dissolved in 4ml of distilled water.

2) 1.318 g of sodium tetrafluoroborate was dissolved in 20 ml of distilled water.

The two solutions were mixed and a white precipitate was immediately formed. The aqueous solution containing the precipitate was extracted several times with dichloromethane. The organic phases were combined, washed several time with water and subsequently dried using magnesium sulfate. The dichloromethane was removed under vacuum and 1.91 g (yield:58.6%) of a white solid was obtained having a melting point of 125.71 °C.

**Ionic liquid 4:**

Two solutions were prepared:

1) 2g of ionic liquid 8 was dissolved in 5ml of distilled water.

2) 826 mg of sodium tetrafluoroborate was dissolved in 4 ml of distilled water.

The two solutions were mixed and a white precipitate was immediately formed. The aqueous solution containing the precipitate was extracted several times with dichloromethane. The organic phases were combined, washed several time with water and subsequently dried using magnesium sulfate. The dichloromethane was removed under vacuum and 231 mg (yield:17%) of a white solid was obtained having a melting point of 164°C.

**Ionic liquid 4:**

Two solutions were prepared:

1) 3g of ionic liquid 9 was dissolved in 4ml of distilled water.

2) 1.2 g of potassium hexafluorophosphate was dissolved in 20 ml of distilled water.

The two solutions were mixed and a white precipitate was immediately formed. The aqueous solution containing the precipitate was extracted several times with dichloromethane. The organic phases were combined, washed several time with water and subsequently dried using magnesium sulfate. The dichloromethane was removed under vacuum and 3.28 g (yield:91%) of a white solid was obtained having a melting point of 163.92°C.

**Ionic liquid 6:**

Two solutions were prepared:

1) 200mg of ionic liquid 1 was dissolved in 4ml of distilled water.

2) 123.9mg of potassium hexafluorophosphate was dissolved in 5 ml of distilled water.

The two solutions were mixed and a white precipitate was immediately formed. The aqueous solution containing the precipitate was extracted several times with dichloromethane. The organic phases were combined, washed several time with water and subsequently dried using magnesium sulfate. The dichloromethane was removed under vacuum and 203mg (yield:86%) of a white solid was obtained having a melting point of 164.62°C.

**Ionic liquid 7:**

Two solutions were prepared:

1) 100mg of ionic liquid 10 was dissolved in 3ml of distilled water.

2) 37.27mg of sodium tetrafluoroborate was dissolved in 2 ml of distilled water.

The two solutions were mixed and a white precipitate was immediately formed. The aqueous solution containing the precipitate was extracted several times with dichloromethane. The organic phases were combined, washed several time with water and subsequently dried using magnesium sulfate. The dichloromethane was removed under vacuum and a white solid was obtained having a melting point of 180°C.

**Ionic liquid 8:**

1.535ml of pyridine have been added to a round bottom flask. 5.5g of 2,6-dibromobenzylbromide dissolved in 5mL of ethyl acetate was subsequently added to the round bottom flask at room temperature. A precipitate was obtained which was filtrated and subsequently washed several times with ethyl acetate. 4.662 g (yield:77.6%) of a white solid was obtained having a melting point of 223°C.

**Ionic liquid 9:**

1.45ml of N-methyl-imidazole have been added to a round bottom flask. 5.3g of 2,6-dibromobenzylbromide dissolved in 4mL of ethyl acetate was subsequently added to the round bottom flask at room temperature. After a few minutes, a precipitate was obtained which was filtrated and subsequently washed several times with ethyl acetate. 5.792 g (yield:49%) of a white solid was obtained having a melting point of 224.86°C.

**Ionic liquid 10:**

245μl of pyridine have been added to a round bottom flask. 1g of 2,3,6-trichlorobenzylbromide dissolved in 4mL of ethyl acetate was subsequently added to the round bottom flask at room temperature. A precipitate was obtained which was filtrated and subsequently washed several times with ethyl acetate. 350 mg (yield:33%) of a white solid was obtained having a melting point of 231 °C.

**Chromatography with a planar stationary phase impregnated with an ionic liquid**

A commercially available polypropylene resin was separated by chromatography on a TLC plate.

The TLC plate was impregnated for 2 minutes with ionic liquid L1 (15 mg/ml of IL1 in ethanol). The plate was subsequently dried before elution. The result of the solution is shown in Figure 1B.

The same experiment was repeated with a TLC plate, wherein the stationary phase was not impregnated with the ionic liquid. The same chromatography conditions were used (i.e. same eluent etc...). The result of the elution is shown in Figure 1A.

It can be seen from Figure 1, that impregnating the stationary phase of the TLC plate with a ionic liquid of the invention allowed a better separation of the polypropylene.

## Claims

1. A method of performing planar chromatography in a method for analyzing, separating, or characterizing polymers selected from polyolefins, polyamides, polycarbonates, poly(hydroxy carboxylic acid), polystyrenes, polyesters, polyethylene terephthalate (PET), polybutylene terephthalate (PBT), polymethylmethacrylate (PMMA), poly(methyl acrylate) (PMA), vinyl polymers, or blends thereof, wherein
the planar stationary phase is a substrate impregnated with an ionic liquid of formula (I), (II), (III) or (IV),

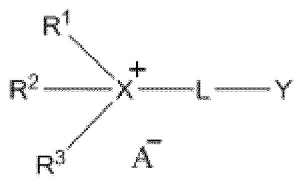

(I)

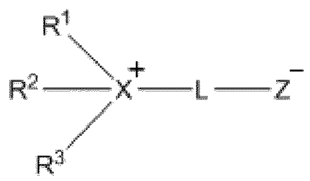

(II)

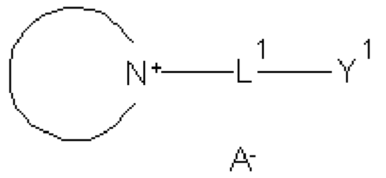

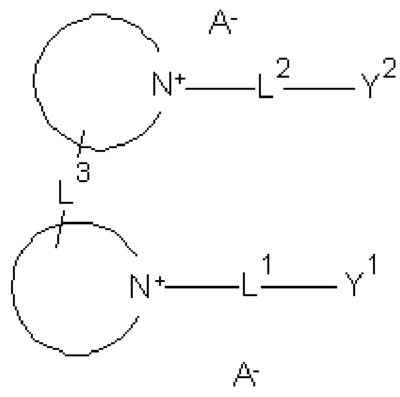

(III) (IV)

wherein

**X** is N, P, or Al;

**$R^1$, $R^2$,** and **$R^3$** are each independently a group selected from $C_{1-10}$alkyl or $C_{6-12}$aryl; each group being optionally substituted with one or more substituents each independently selected from halo, $C_{1-6}$alkyl, $C_{1-6}$alkoxy, cyano, polyhalo$C_{1-6}$alkoxy, $C_{3-7}$cycloalkyl, $C_{6-12}$aryl, oxo, and Het;

**or**

**X** is N, and **$R^1$** is hydrogen or is not present, and **$R^2$,** and **$R^3$,** together with the atom **X** to which they are attached to, form a ring selected from pyridinium, imidazolium, pyrazolium, thiazolium, triazolium, pyrrolium, indolium, tetrazolium, pyrrolidinium, morpholinium pyrimidinium, pyrazinium, pyridazinium, piperazinium, and piperidinium; each optionally substituted with one, two, or three substituents selected from $C_{1-12}$alkyl and $C_{1-12}$alkoxy;

each **ring** comprising the nitrogen atom is independently selected from the group consisting of pyridinium, pyrazinium, pyrrolium, imidazolium, pyrazolium, thiazolium, triazolium, indolium, tetrazolium, pyrimidinium, pyridazinium, piperazinium, and piperidinium; each group being optionally substituted with one, two, or three substituents selected from $C_{1-12}$alkyl and $C_{1-12}$alkoxy;

**L** is $C_{1-12}$alkylene, $C_{3-7}$cycloalkylene, $C_{6-12}$arylene; each of $C_{1-6}$alkylene, $C_{3-7}$cycloalkylene, and $C_{6-12}$arylene, optionally containing 1 to 4 heteroatoms each independently selected from nitrogen, oxygen and sulfur; and each of $C_{1-6}$alkylene, $C_{3-7}$cycloalkylene, and $C_{6-12}$arylene, being optionally substituted one or more substituents each independently selected from $C_{1-6}$alkyl and oxo;

**$L^1$** is $C_{1-12}$alkylene;

**$L^2$** is $C_{1-12}$alkylene;

**$L^3$** is a single bond or $C_{1-12}$alkylene; wherein one carbon of the $C_{1-12}$alkylene is optionally replaced by one or more heteroatoms;

**Y** is hydrogen, halo, $C_{1-10}$alkyl, $C_{6-12}$aryl, $-OR^4$, $-COOR^4$, $-CONR^{5a}R^{5b}$, $-NR^{5a}{}_{R}{}^{5b}$, - $SR^6$, $-SO_2R^7$, $-SiR^8{}_3$, $-OP(O)(OH)_2$, epoxy, or Het; each group being optionally substituted with one or more substituents each independently selected from halo, OH, $-OR^4$, $-COR^4$, $C_{1-6}$alkyl; $C_{6-12}$aryl, $C_{1-6}$alkoxy, oxo, halo$C_{1-6}$alkyl, and halo$C_{1-6}$alkoxy;

**$Y^1$** is $C_{6-12}$aryl substituted with one, two, or three substituents each independently selected from the group consisting of halo, halo$C_{1-6}$alkyl, and halo$C_{1-6}$alkoxy;

**$Y^2$** is $C_{6-12}$aryl substituted with one, two, or three substituents each independently selected from the group consisting of halo, halo$C_{1-6}$alkyl, and halo$C_{1-6}$alkoxy;

**Z** is a carboxylate ($-CO_2$), a sulfonate ($-SO_3$), a sulfinate ($-SO_2$), a phosphorus based anion (such as phosphonate, phosphite, phosphate and the like), an ester monosulfate ($-OSO_3$), or a sulfonamidate ($-NHSO_2$);

**A** is [Br], [$PF_6$], [$BF_4$], [$AsF_6$], [$SbF_6$], [$N(SO_2CF_3)_2$], [$CF_3SO_3$], [$CF_3CO_2$], [$CH_3CO_2$], [$CF_3SO_2$], [$NO_2$], [$NO_3$], [$ClO_4$], [Cl], [I], [HO-CO-O]; and [$AlR^{10}{}_{4-n}R^{11}{}_n$];

**$R^4$** is hydrogen; or a group selected from $C_{1-6}$alkyl; $C_{6-12}$aryl; Het; and $C_{3-7}$cycloalkyl; each group being optionally substituted with one or more substituents each independently selected from $C_{1-6}$alkyl; $C_{3-7}$cycloalkyl, $C_{6-12}$aryl, halo, $C_{1-6}$alkoxy, oxo and Het;

**$R^{5a}$** and **$R^{5b}$** are, each independently, selected from hydrogen, $C_{1-6}$alkyl, and $C_{6-12}$aryl$C_{1-6}$alkyl;

**$R^6$** is hydrogen, $C_{1-6}$alkyl, $C_{6-12}$aryl, $C_{3-7}$cycloalkyl, or $-C(=O)R^9$;

**$R^7$** is hydrogen, $C_{1-6}$alkyl, or $C_{3-7}$cycloalkyl;

**$R^8$** is hydrogen, $C_{1-6}$alkyl optionally substituted with halo, $C_{1-6}$alkoxy, $C_{3-7}$cycloalkyl, or $C_{6-12}$aryl; $C_{3-7}$cycloalkyl; or -$OR^4$;
**$R^9$** is $C_{1-12}$alkyl;
**$R^{10}$** is Cl, Br, F or I or $C_{1-12}$alkyl;
**$R^{11}$** is F, Cl, Br, or I;
**n** is an integer from 0 to 4;
**$C_{6-12}$aryl** as a group or part of a group is phenyl, naphthyl, indanyl, or 1,2,3,4-tetrahydronaphthyl, each of which may be optionally substituted with one, two or three substituents selected from halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, mono- or di$C_{1-6}$alkylaminocarbonyl, azido, mercapto;
**Het** as a group or part of a group is a 5 or 6 membered saturated, partially unsaturated or completely unsaturated heterocyclic ring containing 1 to 4 heteroatoms each independently selected from nitrogen, oxygen and sulfur, said heterocyclic ring being optionally condensed with a benzene ring, and wherein the group Het as a whole may be optionally substituted with one, two or three substituents each independently selected from the group consisting of halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, and mono- or di$C_{1-6}$alkylaminocarbonyl.

**2.** The method of performing planar chromatography according to claim 1, wherein in the ionic liquid of formula (I), (II), (III) or (IV):,

**X** is N,
**$R^1$** is hydrogen or is not present, and **$R^2$,** and **$R^3$,** together with the atom **X** to which they are attached to, form a ring selected from pyridinium, imidazolium, pyrazolium, thiazolium, triazolium, pyrrolium, indolium, tetrazolium, pyrimidinium, pyrazinium, pyridazinium, piperazinium, and piperidinium; each optionally substituted with one, two, or three substituents selected from $C_{1-12}$alkyl and $C_{1-12}$alkoxy;
**L** is $C_{1-6}$alkylene;
**$L^1$** is $C_{1-6}$alkylene;
**$L^2$** is $C_{1-6}$alkylene;
**Y** is $C_{6-12}$aryl substituted with one, two, or three substituents each independently selected from the group consisting of halo, halo$C_{1-6}$alkyl, and halo$C_{1-6}$alkoxy;
**$Y^1$** is $C_{6-10}$aryl substituted with one, two, or three substituents each independently selected from the group consisting of halo, halo$C_{1-6}$alkyl, and halo$C_{1-6}$alkoxy;
**$Y^2$** is $C_{6-10}$aryl substituted with one, two, or three substituents each independently selected from the group consisting of halo, halo$C_{1-6}$alkyl, and halo$C_{1-6}$alkoxy;
**A** is [Br], [$PF_6$], [$BF_4$], [Cl], [$AsF_6$], [$SbF_6$], [$N(SO_2CF_3)_2$], [$CF_3SO_3$], [$CH_3CO_2$], [$CF_3SO_2$], [$NO_2$], [$NO_3$], [$ClO_4$], [I], or [$AlR^{10}_{4-n}R^{11}_n$];
**$Z^-$** is -$CO_2$, -$SO_3$, -$SO_2$, -$PO_2$, -$PO_3$, -$OSO_3$, or -$NHSO_2$.

**3.** The method of performing planar chromatography according to any one of claims 1-2, wherein in the ionic liquid of formula (Ia), or (Ib):

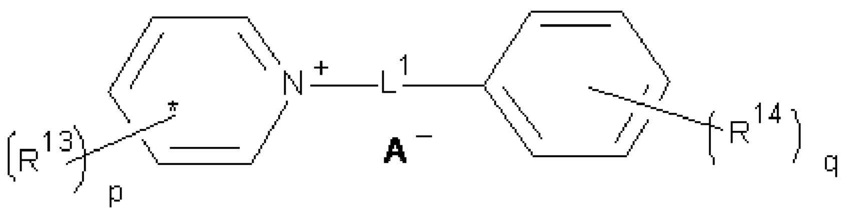

(Ia)

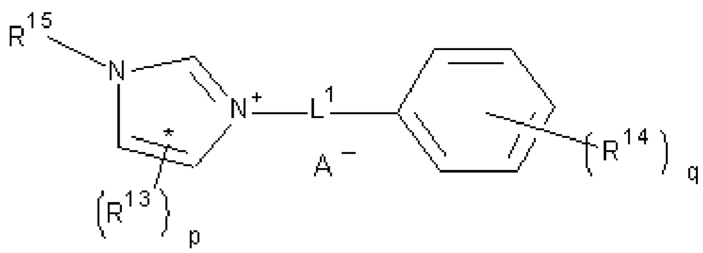

(Ib)

wherein

**A** is selected from the group consisting of [Br], [Cl], [$PF_6$], [$AsF_6$], [$SbF_6$], [$N(SO_2CF_3)_2$], [$BF_4$], [$CF_3SO_3$], [$CH_3CO_2$], [$CF_3SO_2$], [$NO_2$], [$NO_3$], [$ClO_4$], [I], and [$AlR^{10}{}_{4-n}R^{11}{}_n$];
**$R^{10}$** is halo or $C_{1-12}$alkyl; preferably halo or $C_{1-6}$alkyl;
**$R^{11}$** is halo; and
**n** is an integer selected from 0, 1, 2, 3, or 4;
**$L^1$** is $C_{1-6}$alkylene;
each **$R^{13}$** is independently selected from hydrogen, $C_{1-6}$alkyl and $C_{1-6}$alkoxy;
each **$R^{14}$** is independently selected from the group consisting of halo, halo$C_{1-6}$alkyl, and halo$C_{1-6}$alkoxy;
**$R^{15}$** is independently selected from $C_{1-6}$alkyl or hydrogen;
**p** is an integer selected from 1, 2, or 3; and
**q** is an integer selected from 1, 2, or 3.

4. The method of performing planar chromatography according to claim 3, wherein

**A** is selected from the group consisting of [Br], [Cl], [$PF_6$], [$AsF_6$], [$SbF_6$], [$BF_4$], [$CH_3CO_2$], [$CF_3SO_2$], [$NO_2$], [$NO_3$], [$ClO_4$], [I];
**$L^1$** is $C_{1-4}$alkylene;
each **$R^{13}$** is independently selected from hydrogen, $C_{1-4}$alkyl and $C_{1-4}$alkoxy; preferably hydrogen
each **$R^{14}$** is independently selected from chloro, fluoro, and bromo, preferably chloro or fluoro,
**$R^{15}$** is independently selected from $C_{1-4}$alkyl or hydrogen ;
**p** is 1, 2 or 3, and
**q** is 1, 2, or 3.

5. The method of performing planar chromatography according to any one of claims 1-4, wherein the substrate comprises silica based normal and reverse-phase resin optionally derivatized with alkyl groups or aromatic groups, preferably silica gel 60F254.

6. The method of performing planar chromatography according to any one of claims 1-5, wherein the chromatography is performed at a working temperature between 30°C and 250°C.

7. The method of performing planar chromatography according to any one of claims 1-6, wherein the chromatography is performed with a temperature gradient.

8. The method of performing planar chromatography according to claim 7, wherein the temperature gradient is greater than, or equal to 1 °C per minute.

9. The method of performing planar chromatography according to any one of claims 1-8, wherein the chromatography is performed using a mobile phase selected from the group comprising toluene, xylene, tetrahydrofuran, $C_4$-$C_{20}$ branched or non-branched alkane, supercritical carbon dioxide, trichlorobenzene, ethyl acetate, dimethylsulfoxide, dimethylformamide, an ionic liquid of formula (I) or (II) as defined in any one of claims 1-2, and mixtures thereof; optionally in the presence of a co-eluent selected from the group comprising an alcohol and an ionic liquid of formula (I) or (II), as defined in any one of claims 1-2.

10. The method of performing planar chromatography according to any one of claims 1-9, wherein the chromatography is performed under external pressure by applying either i) a neutral gas under pressure applied to a membrane on

the planar stationary phase; ii) a pneumatic pillow or metallic membrane on the planar stationary phase; or iii) a pneumatic press on the planar stationary phase.

**11.** The method of performing planar chromatography according to any one of claims 1-10, wherein the chromatography is subjected to a gradient elution and comprises at least two subsequent chromatography steps with progressively decreasing migration distances of the mobile phase on the planar chromatography plate, wherein said gradient elution comprises increasing the elution strength of the mobile phase between two subsequent chromatography steps, said steps being performed on the same planar chromatography plate.

**12.** The method of performing planar chromatography according to any one of claims 1-11, wherein planar chromatography is selected from a Thin-layer chromatography (TLC), High Performance Thin-Layer Chromatography (HPTLC), and Overpressurized Layer Chromatography (OPLC).

**13.** The method of performing planar chromatography according to any one of claims 1-12, wherein planar chromatography is Overpressurized Layer Chromatography (OPLC).

**14.** The method of performing planar chromatography according to any one of claims 1-13, wherein the polymer is a polyolefin.

**15.** The method of performing planar chromatography according to any one of claims 1-14, wherein the polymer is selected from polyethylene, polypropylene, polystyrene, and polylactic acid.

**Patentansprüche**

**1.** Verfahren zum Durchführen einer planaren Chromatographie im Rahmen eines Verfahrens zum Analysieren, Trennen oder Charakterisieren von Polymeren, die aus Polyolefinen, Polyamiden, Polycarbonaten, Poly(hydroxycarbonsäure), Polystyrolen, Polyestern, Polyethylenterephthalat (PET), Polybutylenterephthalat (PBT), Polymethylmethacrylat (PMMA), Poly(methylacrylat) (PMA), Vinylpolymeren oder Mischungen davon ausgewählt sind, wobei die planare stationäre Phase ein Substrat ist, das imprägniert ist mit einer ionischen Flüssigkeit der Formel (I), (II), (III) oder (IV)

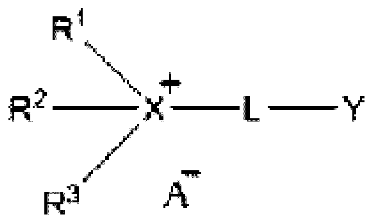

(I)

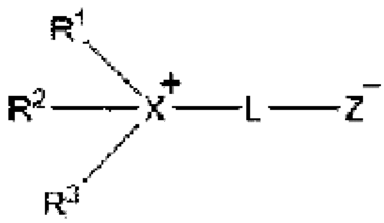

(II)

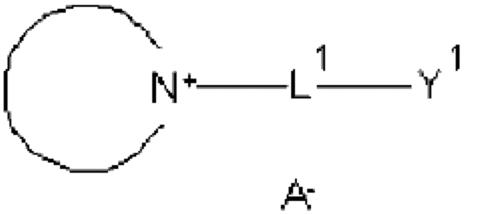 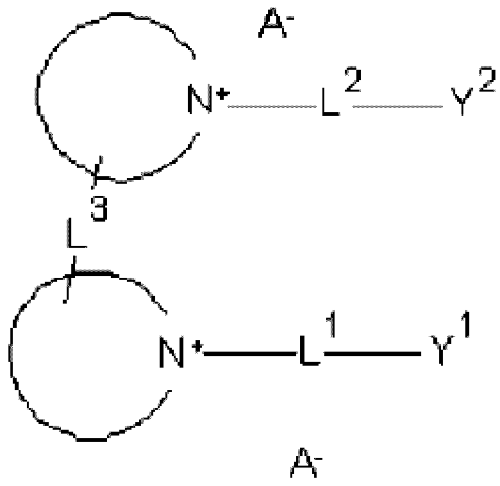

(III) (IV)

wobei:

**X** N, P oder Al ist;
**R1, R2** und **R3** jeweils unabhängig eine Gruppe sind, die aus $C_{1-10}$-Alkyl oder $C_{6-12}$-Aryl ausgewählt ist; wobei jede Gruppe optional durch einen oder mehrere Substituenten substituiert ist, die jeweils unabhängig aus Halo, $C_{1-6}$-Alkyl, $C_{1-6}$-Alkoxy, Cyano, Polyhalo-$C_{1-6}$-alkoxy, $C_{3-7}$-Cycloalkyl, $C_{6-12}$-Aryl, Oxo und Het ausgewählt sind;
**oder**
**X** N ist, und **R1** Wasserstoff oder nicht vorhanden ist, und **R2** und **R3** gemeinsam mit dem Atom **X,** an das sie angehaftet sind, einen Ring bilden, der aus Pyridinium, Imidazolium, Pyrazolium, Thiazolium, Triazolium, Pyrrolium, Indolium, Tetrazolium, Pyrrolidinium, Morpholinium, Pyrimidinium, Pyrazinium, Pyridazinium, Piperazinium und Piperidinium ausgewählt ist; jeweils optional durch einen, zwei oder drei Substituenten substituiert, die aus $C_{1-12}$-Alkyl und $C_{1-12}$-Alkoxy ausgewählt sind;
jeder **Ring,** der das Stickstoffatom umfasst, unabhängig aus der Gruppe ausgewählt ist, bestehend aus Pyridinium, Pyrazinium, Pyrrolium, Imidazolium, Pyrazolium, Thiazolium, Triazolium, Indolium, Tetrazolium, Pyrimidinium, Pyridazinium, Piperazinium und Piperidinium; wobei jede Gruppe optional durch einen, zwei oder drei Substituenten substituiert ist, die aus $C_{1-12}$-Alkyl und $C_{1-12}$-Alkoxy ausgewählt sind;
**L** $C_{1-12}$-Alkylen, $C_{3-7}$-Cycloalkylen, $C_{6-12}$-Arylen ist; $C_{1-6}$-Alkylen, $C_{3-7}$-Cycloalkylen und $C_{6-12}$-Arylen jeweils optional 1 bis 4 Heteroatome enthalten, die jeweils unabhängig aus Stickstoff, Sauerstoff und Schwefel ausgewählt sind; und $C_{1-6}$-Alkylen, $C_{3-7}$-Cycloalkylen und $C_{6-12}$-Arylen jeweils optional durch einen oder mehr Substituenten substituiert sind, die jeweils unabhängig aus $C_{1-6}$-Alkyl und Oxo ausgewählt sind;
**L1** $C_{1-12}$-Alkylen ist;
**L2** $C_{1-12}$-Alkylen ist;
**L3** eine Einfachbindung oder $C_{1-12}$-Alkylen ist; wobei ein Kohlenstoff des $C_{1-12}$-Alkylens optional durch ein oder mehrere Heteroatome ersetzt ist;
**Y** Wasserstoff, Halo, $C_{1-10}$-Alkyl, $C_{6-12}$-Aryl, $-OR^4$, $-COOR^4$, $-CONR^{5a}R^{5b}$, $-NR^{5a}R^{5b}$, $-SR^6$, $-SO_2R^7$, $-SiR^8_3$, $-OP(O)(OH)_2$, Epoxid oder Het ist; wobei jede Gruppe optional durch einen oder mehrere Substituenten substituiert ist, die unabhängig aus Halo, OH, $-OR^4$, $-COR^4$, $C_{1-6}$-Alkyl; $C_{6-12}$-Aryl, $C_{1-6}$-Alkoxy, Oxo, Halo-$C_{1-6}$-alkyl und Halo-$C_{1-6}$-alkoxy ausgewählt sind;
**Y1** $C_{6-12}$-Aryl ist, das durch einen, zwei oder drei Substituenten substituiert ist, die jeweils unabhängig aus der Gruppe ausgewählt sind, bestehend aus Halo, Halo-$C_{1-6}$-alkyl und Halo-$C_{1-6}$-alkoxy;
**Y2** $C_{6-12}$-Aryl ist, das durch ein, zwei oder drei Substituenten substituiert ist, die jeweils unabhängig aus der Gruppe ausgewählt sind, bestehend aus Halo, Halo-$C_{1-6}$-Alkyl und Halo-$C_{1-6}$-alkoxy;
**Z** ein Carboxylat ($-CO_2$), ein Sulfonat ($-SO_3$), ein Sulfinat ($-SO_2$), ein phosphorbasiertes Anion (z. B. Phosphonat, Phosphit, Phosphat und dergleichen), ein Estermonosulfat ($-OSO_3$) oder ein Sulfonamidat ($-NHSO_2$) ist;
**A** [Br], $[PF_6]$, $[BF_4]$, $[AsF_6]$, $[SbF_6]$, $[N(SO_2CF_3)_2]$, $[CF_3SO_3]$, $[CF_3CO_2]$, $[CH_3CO_2]$, $[CF_3SO_2]$, $[NO_2]$, $[NO_3]$, $[ClO_4]$, [Cl], [I], [HO-CO-O]; und $[AlR^{10}_{4-n}R^{11}_n]$ ist;
**R4** Wasserstoff; oder eine Gruppe ist, die aus $C_{1-6}$-Alkyl; $C_{6-12}$-Aryl; Het; und $C_{3-7}$-Cycloalkyl ausgewählt ist;

wobei jede Gruppe optional durch einen oder mehrere Substituenten substituiert ist, die unabhängig aus $C_{1-6}$-Alkyl; $C_{3-7}$-Cycloalkyl, $C_{6-12}$-Aryl, Halo, $C_{1-6}$-Alkoxy, Oxo und Het ausgewählt sind;

**$R^{5a}$** und **$R^{5b}$** jeweils unabhängig aus Wasserstoff, $C_{1-6}$-Alkyl und $C_{6-12}$-Aryl-$C_{1-6}$-alkyl ausgewählt sind;

**$R^6$** Wasserstoff, $C_{1-6}$-Alkyl, $C_{6-12}$-Aryl, $C_{3-7}$-Cycloalkyl oder-C(=O)$R^9$ ist;

**$R^7$** Wasserstoff, $C_{1-6}$-Alkyl oder $C_{3-7}$-Cycloalkyl ist;

**$R^8$** Wasserstoff, $C_{1-6}$-Alkyl, optional substituiert durch Halo, $C_{1-6}$-Alkoxy, $C_{3-7}$-Cycloalkyl oder $C_{6-12}$-Aryl; $C_{3-7}$-Cycloalkyl; oder -$OR^4$ ist;

**$R^9$** $C_{1-12}$-Alkyl ist;

**$R^{10}$** Cl, Br, F oder I oder $C_{1-12}$-Alkyl ist;

**$R^{11}$** F, Cl, Br oder I ist;

**n** eine ganze Zahl von 0 bis 4 ist;

**$C_{6-12}$-Aryl** als Gruppe oder Teil einer Gruppe Phenyl, Naphthyl, Indanyl oder 1,2,3,4-Tetrahydronaphthyl ist, die jeweils optional durch einen, zwei oder drei Substituenten substituiert sein können, die aus Halo, $C_{1-6}$-Alkyl, Polyhalo-$C_{1-6}$-alkyl, Hydroxy, $C_{1-6}$-Alkoxy, Polyhalo-$C_{1-6}$-alkoxy, $C_{1-6}$-Alkoxy-$C_{1-6}$-alkyl, Carboxyl, $C_{1-6}$-Alkylcarbonyl, $C_{1-6}$-Alkoxycarbonyl, Cyano, Nitro, Amino, Mono- oder Di-$C_{1-6}$-alkylamino, Aminocarbonyl, Mono- oder Di-$C_{1-6}$-alkylaminocarbonyl, Azido, Mercapto ausgewählt sind;

**Het** als Gruppe oder Teil einer Gruppe ein 5- oder 6-gliedriger, gesättigter, teilweise ungesättigter oder gänzlich ungesättigter, heterocyclischer Ring ist, der 1 bis 4 Heteroatome enthält, die jeweils unabhängig aus Stickstoff, Sauerstoff und Schwefel ausgewählt sind, wobei der heterocyclische Ring optional mit einem Benzolring kondensiert ist, und wobei die Gruppe Het als Ganzes optional durch einen, zwei oder drei Substituenten substituiert sein kann, die jeweils unabhängig aus der Gruppe ausgewählt sind, bestehend aus Halo, $C_{1-6}$-Alkyl, Polyhalo-$C_{1-6}$-alkyl, Hydroxy, $C_{1-6}$-Alkoxy, Polyhalo-$C_{1-6}$-alkoxy, $C_{1-6}$-Alkoxy-$C_{1-6}$-alkyl, Carboxyl, $C_{1-6}$-Alkylcarbonyl, $C_{1-6}$-Alkoxycarbonyl, Cyano, Nitro, Amino, Mono- oder Di-$C_{1-6}$-Alkylamino, Aminocarbonyl und Mono- oder Di-$C_{1-6}$-Alkylaminocarbonyl.

**2.** Verfahren zum Durchführen einer planaren Chromatographie nach Anspruch 1, wobei bei der ionischen Flüssigkeit der Formel (I), (II), (III) oder (IV):

**X** N ist,

**$R^1$** Wasserstoff oder nicht vorhanden ist, und **$R^2$** und **$R^3$** gemeinsam mit dem Atom X, an das sie angehaftet sind, einen Ring bilden, der aus Pyridinium, Imidazolium, Pyrazolium, Thiazolium, Triazolium, Pyrrolium, Indolium, Tetrazolium, Pyrimidinium, Pyrazinium, Pyridazinium, Piperazinium und Piperidinium ausgewählt ist; jeweils optional durch einen, zwei oder drei Substituenten substituiert, die aus $C_{1-12}$-Alkyl und $C_{1-12}$-Alkoxy ausgewählt sind;

**L** $C_{1-6}$-Alkylen ist;

**$L^1$** $C_{1-6}$-Alkylen ist;

**$L^2$** $C_{1-6}$-Alkylen ist;

**Y** $C_{6-12}$-Aryl ist, das durch einen, zwei oder drei Substituenten substituiert ist, die jeweils unabhängig aus der Gruppe ausgewählt sind, bestehend aus Halo, Halo-$C_{1-6}$-Alkyl und Halo-$C_{1-6}$-Alkoxy;

**$Y^1$** $C_{6-10}$-Aryl ist, das durch ein, zwei oder drei Substituenten substituiert ist, die jeweils unabhängig aus der Gruppe ausgewählt sind, bestehend aus Halo, Halo-$C_{1-6}$-Alkyl und Halo-$C_{1-6}$-Alkoxy;

**$Y^2$** $C_{6-10}$-Aryl ist, das durch ein, zwei oder drei Substituenten substituiert ist, die jeweils unabhängig aus der Gruppe ausgewählt sind, bestehend aus Halo, Halo-$C_{1-6}$-Alkyl und Halo-$C_{1-6}$-Alkoxy;

**A** [Br], [$PF_6$], [$BF_4$], [Cl], [$AsF_6$], [$SbF_6$], [$N(SO_2CF_3)_2$], [$CF_3SO_3$], [$CH_3CO_2$], [$CF_3SO_2$], [$NO_2$], [$NO_3$], [$CLO_4$], [I] oder [$AlR^{10}_{4-n}R^{11}_n$] ist;

**$Z^-$** -$CO_2$, -$SO_3$, -$SO_2$, -$PO_2$, -$PO_3$, -$OSO_3$ oder -$NHSO_2$ ist.

**3.** Verfahren zum Durchführen einer planaren Chromatographie nach einem der Ansprüche 1 bis 2, wobei bei der ionischen Flüssigkeit der Formel (Ia) oder (Ib):

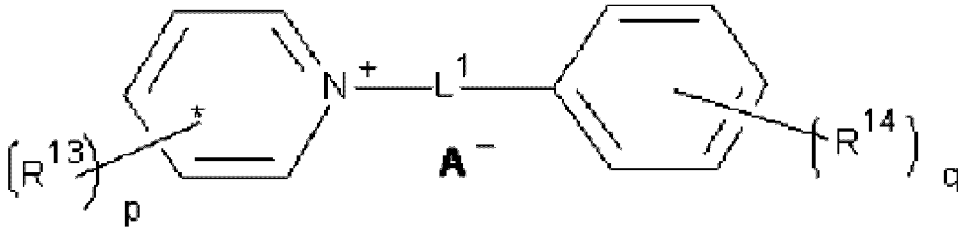

(Ia)

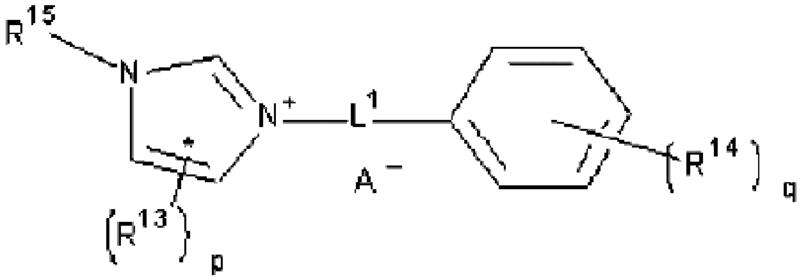

(Ib)

wobei:

**A** aus der Gruppe ausgewählt ist, bestehend aus [Br], [Cl], [$PF_6$], [$AsF_6$], [$SbF_6$], [$N(SO_2CF_3)_2$], [$BF_4$], [$CF_3SO_3$], [$CH_3CO_2$], [$CF_3SO_2$], [$NO_2$], [$NO_3$], [$ClO_4$], [I] und [$AlR^{10}{}_{4-n}R^{11}{}_n$];
**$R^{10}$** Halo oder $C_{1-12}$-Alkyl ist; vorzugsweise Halo oder $C_{1-6}$-Alkyl;
**$R^{11}$** Halo ist; und
**n** eine ganze Zahl ist, die aus 0, 1, 2, 3, oder 4 ausgewählt ist;
**$L^1$** $C_{1-6}$-Alkylen ist;
**$R^{13}$** jeweils unabhängig aus Wasserstoff, $C_{1-6}$-Alkyl und $C_{1-6}$-Alkoxy ausgewählt ist;
**$R^{14}$** jeweils unabhängig aus der Gruppe ausgewählt ist, bestehend aus Halo, Halo-$C_{1-6}$-alkyl und Halo-$C_{1-6}$-alkoxy;
**$R^{15}$** unabhängig aus $C_{1-6}$-Alkyl oder Wasserstoff ausgewählt ist;
**p** eine ganze Zahl ist, die aus 1, 2 oder 3 ausgewählt ist; und
**q** eine ganze Zahl ist, die aus 1, 2 oder 3 ausgewählt ist.

**4.** Verfahren zum Durchführen einer planaren Chromatographie nach Anspruch 3, wobei

**A** aus der Gruppe ausgewählt ist, bestehend aus [Br], [Cl], [$PF_6$], [$AsF_6$], [$SbF_6$], [$BF_4$], [$CH_3CO_2$], [$CF_3SO_2$], [$NO_2$], [$NO_3$], [$ClO_4$], [I];
**$L^1$** $C_{1-4}$-Alkylen ist;
**$R^{13}$** jeweils unabhängig aus Wasserstoff, $C_{1-4}$-Alkyl und $C_{1-4}$-Alkoxy ausgewählt ist; vorzugsweise Wasserstoff,
**$R^{14}$** jeweils unabhängig aus Chlor, Fluor und Brom ausgewählt ist, vorzugsweise Chlor oder Fluor,
**$R^{15}$** unabhängig aus $C_{1-4}$-Alkyl oder Wasserstoff ausgewählt ist;
**p** 1, 2 oder 3 ist, und
**q** 1, 2 oder 3 ist.

**5.** Verfahren zum Durchführen einer planaren Chromatographie nach einem der Ansprüche 1 bis 4, wobei das Substrat siliciumdioxidbasiertes normales und Umkehrphasen-Harz umfasst, das optional mit Alkylgruppen oder aromatischen Gruppen derivatisiert ist, vorzugsweise Siliciumdioxidgel 60F254.

**6.** Verfahren zum Durchführen einer planaren Chromatographie nach einem der Ansprüche 1 bis 5, wobei die Chromatographie bei einer Arbeitstemperatur zwischen 30 °C und 250 °C durchgeführt wird.

**7.** Verfahren zum Durchführen einer planaren Chromatographie nach einem der Ansprüche 1 bis 6, wobei die Chromatographie mit einem Temperaturgradienten durchgeführt wird.

**8.** Verfahren zum Durchführen einer planaren Chromatographie nach Anspruch 7, wobei der Temperaturgradient größer gleich 1 °C pro Minute ist.

**9.** Verfahren zum Durchführen einer planaren Chromatographie nach einem der Ansprüche 1 bis 8, wobei die Chromatographie unter Verwendung einer mobilen Phase, die aus der Gruppe ausgewählt ist, umfassend Toluol, Xylol, Tetrahydrofuran, verzweigtem oder unverzweigten $C_4$-$C_{20}$-Alkan, superkritischem Kohlendioxid, Trichlorbenzol, Ethylacetat, Dimethylsulfoxid, Dimethylformamid, eine ionische Flüssigkeit der Formel (I) oder (II), wie in einem der Ansprüche 1 bis 2 definiert, und Mischungen davon; optional in der Gegenwart eines Coelutionsmittels durchgeführt wird, das aus der Gruppe ausgewählt ist, umfassend einen Alkohol und eine ionische Flüssigkeit der Formel (I) oder (II), wie in einem der Ansprüche 1 bis 2 definiert.

**10.** Verfahren zum Durchführen einer planaren Chromatographie nach einem der Ansprüche 1 bis 9, wobei die Chromatographie unter externem Druck durchgeführt wird, indem entweder i) ein neutrales Gas, das unter Druck auf eine Membran auf der planaren stationären Phase angelegt wird; ii) ein pneumatisches Polster oder eine metallische Membran auf die planare stationäre Phase; oder iii) ein pneumatischer Druck auf die planare stationäre Phase angelegt wird.

**11.** Verfahren zum Durchführen einer planaren Chromatographie nach einem der Ansprüche 1 bis 10, wobei die Chromatographie einer Gradientenelution ausgesetzt wird und zumindest zwei aufeinander folgende Chromatographieschritte mit progressiv abnehmenden Wanderungsweiten der mobilen Phase auf der planaren Chromatographieplatte umfasst, wobei die Gradientenelution das Erhöhen der Elutionsstärke der mobilen Phase zwischen zwei aufeinander folgenden Chromatographieschritte umfasst, wobei die Schritte auf der gleichen planaren Chromatographieplatte durchgeführt werden.

**12.** Verfahren zum Durchführen einer planaren Chromatographie nach einem der Ansprüche 1 bis 11, wobei die planare Chromatographie aus einer Dünnschichtchromatographie (TLC), Hochleistungs-Dünnschichtchromatographie (HPTLC) und Überdruck-Schichtchromatographie (OPLC) ausgewählt ist.

**13.** Verfahren zum Durchführen einer planaren Chromatographie nach einem der Ansprüche 1 bis 12, wobei die planare Chromatographie Überdruck-Schichtchromatographie (OPLC) ist.

**14.** Verfahren zum Durchführen einer planaren Chromatographie nach einem der Ansprüche 1 bis 13, wobei das Polymer ein Polyolefin ist.

**15.** Verfahren zum Durchführen einer planaren Chromatographie nach einem der Ansprüche 1 bis 14, wobei das Polymer aus Polyethylen, Polypropylen, Polystyrol und Polymilchsäure ausgewählt ist.

## Revendications

**1.** Procédé pour effectuer une chromatographie de surface dans un procédé pour analyser, séparer ou caractériser des polymères choisis parmi les polyoléfines, les polyamides, les polycarbonates, le poly(acide hydroxycarboxylique), les polystyrènes, les polyesters, le poly(téréphtalate d'éthylène) (PET), le poly(téréphtalate de butylène) (PBT), le poly(méthacrylate de méthyle) (PMMA), le poly(acrylate de méthyle) (PMA), les polymères vinyliques, et leurs mélanges, dans lequel
la phase stationnaire plane est un substrat imprégné par un liquide ionique de formule (I), (II), (III) ou (IV),

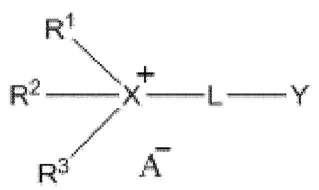
(I)

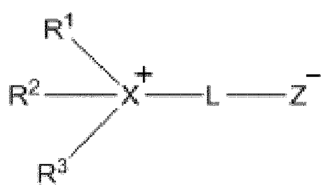
(II)

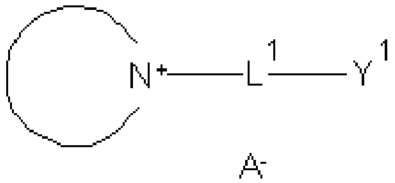

(III)

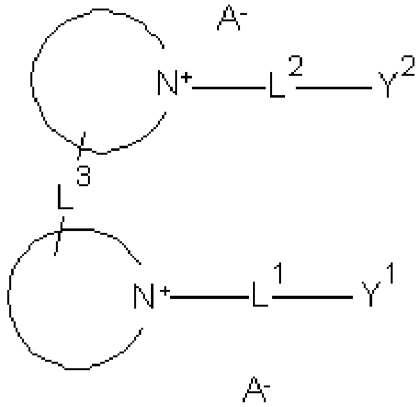

(IV)

où

X est N, P, ou Al ;
chacun de $R^1$, $R^2$ et $R^3$ est indépendamment un groupe choisi parmi alkyle en $C_1$ à $C_{10}$ et aryle en $C_6$ à $C_{12}$ ; chaque groupe étant éventuellement substitué par un ou plusieurs substituants, chacun indépendamment choisi parmi halogéno, alkyle en $C_1$ à $C_6$, alcoxy en $C_1$ à $C_6$, cyano, polyhalogénoalcoxy en $C_1$ à $C_6$, cycloalkyle en $C_3$ à $C_7$, aryle en $C_6$ à $C_{12}$, oxo, et Het ;
ou bien
X est N et $R^1$ est l'hydrogène ou est absent, et $R^2$ et $R^3$, ensemble avec l'atome X auquel ils sont rattachés, forment un cycle choisi parmi pyridinium, imidazolium, pyrazolium, thiazolium, triazolium, pyrrolium, indolium, tétrazolium, pyrrolidinium, morpholinium, pyrimidinium, pyrazinium, pyridazinium, pipérazinium et pipéridinium ; chacun étant éventuellement substitué par un, deux ou trois substituants choisis parmi alkyle en $C_1$ à $C_{12}$ et alcoxy en $C_1$ à $C_{12}$ ;
chaque cycle comprenant l'atome d'azote est indépendamment choisi dans l'ensemble constitué par pyridinium, pyrazinium, pyrrolium, imidazolium, pyrazolium, thiazolium, triazolium, indolium, tétrazolium, pyrimidinium, pyridazinium, pipérazinium, et pipéridinium ; chaque groupe étant éventuellement substitué par un, deux ou trois substituants choisis parmi alkyle en $C_1$ à $C_{12}$ et alcoxy en $C_1$ à $C_{12}$ ;
L est un alkylène en $C_1$ à $C_{12}$, cycloalkylène en $C_3$ à $C_7$, arylène en $C_6$ à $C_{12}$ ; chaque alkylène en $C_1$ à $C_6$, cycloalkylène en $C_3$ à $C_7$, et arylène en $C_6$ à $C_{12}$ contenant éventuellement 1 à 4 hétéroatomes, chacun indépendamment choisi parmi l'azote, l'oxygène et le soufre ; et chaque alkylène en $C_1$ à $C_6$, cycloalkylène en $C_3$ à $C_7$, et arylène en $C_6$ à $C_{12}$ étant éventuellement substitué par un ou plusieurs substituants, chacun indépendamment choisi parmi alkyle en $C_1$ à $C_6$ et oxo ;
$L^1$ est un alkylène en $C_1$ à $C_{12}$ ;
$L^2$ est un alkylène en $C_1$ à $C_{12}$ ;
$L^3$ est une liaison simple ou un alkylène en $C_1$ à $C_{12}$ ; un carbone de l'alkylène en $C_1$ à $C_{12}$ étant éventuellement remplacé par un ou plusieurs hétéroatomes ;
Y est l'hydrogène ou un halogène, alkyle en $C_1$ à $C_{10}$, aryle en $C_6$ à $C_{12}$, $-OR^4$, $-COOR^4$, $-CONR^{5a}R^{5b}$, $-NR^{5a}R^{5b}$, $-SR^6$, $-SO_2R^7$, $-SiR^8_3$, - $OP(O)(OH)_2$, époxy, ou Het ; chaque groupe étant éventuellement substitué par un ou plusieurs substituants, chacun indépendamment choisi parmi halogéno, OH, $-OR^4$, $-COR^4$, alkyle en $C_1$ à $C_6$ ; aryle en $C_6$ à $C_{12}$, alcoxy en $C_1$ à $C_6$, oxo, halogénoalkyle en $C_1$ à $C_6$, et halogénoalcoxy en $C_1$ à $C_6$ ;
$Y^1$ est un aryle en $C_6$ à $C_{12}$ substitué par un, deux ou trois substituants, chacun indépendamment choisi dans l'ensemble constitué par halogéno, halogénoalkyle en $C_1$ à $C_6$, et halogénoalcoxy en $C_1$ à $C_6$ ;
$Y^2$ est un aryle en $C_6$ à $C_{12}$ substitué par un, deux ou trois substituants, chacun indépendamment choisi dans l'ensemble constitué par halogéno, halogénoalkyle en $C_1$ à $C_6$, et halogénoalcoxy en $C_1$ à $C_6$ ;
Z est un carboxylate ($-CO_2$), un sulfonate ($-SO_3$), un sulfinate ($-SO_2$), un anion à base de phosphore (tel que phosphonate, phosphite, phosphate et analogue), un ester monosulfate ($-OSO_3$), ou un sulfonamidate ($-NHSO_2$) ;
A est [Br], $[PF_6]$, $[BF_4]$, $[AsF_6]$, $[SbF_6]$, $[N(SO_2CF_3)_2]$, $[CF_3SO_3]$, $[CF_3CO_2]$, $[CH_3CO_2]$, $[CF_3SO_2]$, $[NO_2]$, $[NO_3]$, $[ClO_4]$, [Cl], [I], [HO-CO-O] ; et $[AlR^{10}_{4-n}R^{11}_n]$ ;
$R^4$ est l'hydrogène ; ou un groupe choisi parmi alkyle en $C_1$ à $C_6$ ; aryle en $C_6$ à $C_{12}$ ; Het ; et cycloalkyle en $C_3$ à $C_7$ ; chaque groupe étant éventuellement substitué par un ou plusieurs substituants, chacun indépendamment choisi parmi alkyle en $C_1$ à $C_6$ ; cycloalkyle en $C_3$ à $C_7$, aryle en $C_6$ à $C_{12}$, halogéno, alcoxy en $C_1$ à $C_6$, oxo et Het ;

chacun de $R^{5a}$ et $R^{5b}$ est indépendamment choisi parmi l'hydrogène, alkyle en $C_1$ à $C_6$, et (aryle en $C_6$ à $C_{12}$)-alkyle en $C_1$ à $C_6$ ;

$R^6$ est l'hydrogène ou un alkyle en $C_1$ à $C_6$, aryle en $C_6$ à $C_{12}$, cycloalkyle en $C_3$ à $C_7$ ou -C(=O)$R^9$ ;

$R^7$ est l'hydrogène ou un alkyle en $C_1$ à $C_6$ ou cycloalkyle en $C_3$ à $C_7$ ;

$R^8$ est l'hydrogène ou un alkyle en $C_1$ à $C_6$ éventuellement substitué par halogéno, alcoxy en $C_1$ à $C_6$, cycloalkyle en $C_3$ à $C_7$, ou aryle en $C_6$ à $C_{12}$ ; cycloalkyle en $C_3$ à $C_7$ ; ou -$OR^4$ ;

$R^9$ est un alkyle en $C_1$ à $C_{12}$ ;

$R^{10}$ est Cl, Br, F, I ou un alkyle en $C_1$ à $C_{12}$ ;

$R^{11}$ est F, Cl, Br ou I ;

n est un entier de 0 à 4 ;

l'aryle en $C_6$ à $C_{12}$, en tant que groupe ou en tant que partie d'un groupe, est phényle, naphtyle, indanyle, ou 1,2,3,4-tétrahydronaphtyle, chacun pouvant être éventuellement substitué par un, deux ou trois substituants choisis parmi halogéno, alkyle en $C_1$ à $C_6$, polyhalogénoalkyle en $C_1$ à $C_6$, hydroxy, alcoxy en $C_1$ à $C_6$, polyhalogénoalcoxy en $C_1$ à $C_6$, (alcoxy en $C_1$ à $C_6$)-alkyle en $C_1$ à $C_6$, carboxyle, (alkyle en $C_1$ à $C_6$)carbonyle, (alcoxy en $C_1$ à $C_6$)-carbonyle, cyano, nitro, amino, mono- ou di-(alkyle en $C_1$ à $C_6$)-amino, aminocarbonyle, mono- ou di-(alkyle en $C_1$ à $C_6$)aminocarbonyle, azido, mercapto ;

Het, en tant que groupe ou en tant que partie d'un groupe, est un hétérocycle à 5 ou 6 chaînons saturé, partiellement insaturé ou entièrement insaturé, contenant 1 à 4 hétéroatomes, chacun indépendamment choisi parmi l'azote, l'oxygène et le soufre ; ledit hétérocycle étant éventuellement condensé à un cycle benzène, et le groupe Het dans son entier pouvant être éventuellement substitué par un, deux ou trois substituants, chacun indépendamment choisi dans l'ensemble constitué par halogéno, alkyle en $C_1$ à $C_6$, polyhalogénoalkyle en $C_1$ à $C_6$, hydroxy, alcoxy en $C_1$ à $C_6$, polyhalogénoalcoxy en $C_1$ à $C_6$, (alcoxy en $C_1$ à $C_6$)-alkyle en $C_1$ à $C_6$, carboxyle, (alkyle en $C_1$ à $C_6$)carbonyle, (alcoxy en $C_1$ à $C_6$)-carbonyle, cyano, nitro, amino, mono- ou di-(alkyle en $C_1$ à $C_6$)-amino, aminocarbonyle, et mono- ou di-(alkyle en $C_1$ à $C_6$)aminocarbonyle.

**2.** Procédé pour effectuer une chromatographie de surface selon la revendication 1, dans lequel, dans le liquide ionique de formule (I), (II), (III) ou (In) :

X est N,

$R^1$ est l'hydrogène ou est absent, et $R^2$ et $R^3$, ensemble avec l'atome X auquel ils sont rattachés, forment un cycle choisi parmi pyridinium, imidazolium, pyrazolium, thiazolium, triazolium, pyrrolium, indolium, tétrazolium, pyrimidinium, pyrazinium, pyridazinium, pipérazinium, et pipéridinium ;

chacun étant éventuellement substitué par un, deux ou trois substituants choisis parmi alkyle en $C_1$ à $C_{12}$ et alcoxy en $C_1$ à $C_{12}$ ;

L est un alkylène en $C_1$ à $C_6$ ;

$L^1$ est un alkylène en $C_1$ à $C_6$ ;

$L^2$ est un alkylène en $C_1$ à $C_6$ ;

Y est un aryle en $C_6$ à $C_{12}$ substitué par un, deux ou trois substituants, chacun indépendamment choisi dans l'ensemble constitué par halogéno, halogénoalkyle en $C_1$ à $C_6$, et
halogénoalcoxy en $C_1$ à $C_6$ ;

$Y^1$ est un aryle en $C_6$ à $C_{10}$ substitué par un, deux ou trois substituants, chacun indépendamment choisi dans l'ensemble constitué par halogéno, halogénoalkyle en $C_1$ à $C_6$, et
halogénoalcoxy en $C_1$ à $C_6$ ;

$Y^2$ est un aryle en $C_6$ à $C_{10}$ substitué par un, deux ou trois substituants, chacun indépendamment choisi dans l'ensemble constitué par halogéno, halogénoalkyle en $C_1$ à $C_6$, et
halogénoalcoxy en $C_1$ à $C_6$ ;

A est [Br], $[PF_6]$, $[BF_4]$, [Cl], $[AsF_6]$, $[SbF_6]$, $[N(SO_2CF_3)_2]$, $[CF_3SO_3]$, $[CH_3CO_2]$, $[CF_3SO_2]$, $[NO_2]$, $[NO_3]$, $[ClO_4]$, [I], ou
$[AlR^{10}{}_{4-n}R^{11}{}_n]$ ;

Z est $-CO_2$, $-SO_3$, $-SO_2$, $-PO_2$, $-PO_3$, $-OSO_3$, ou $-NHSO_2$.

**3.** Procédé pour effectuer une chromatographie de surface selon l'une quelconque des revendications 1 et 2, dans lequel le liquide ionique est de formule (Ia) ou (Ib) :

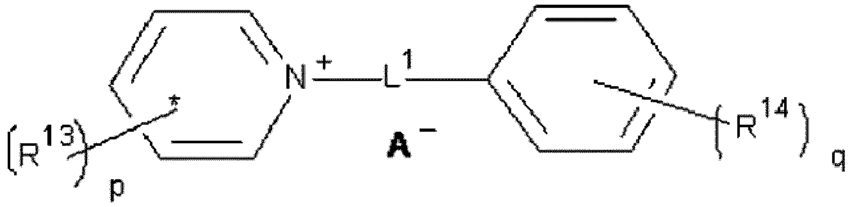

(Ia)

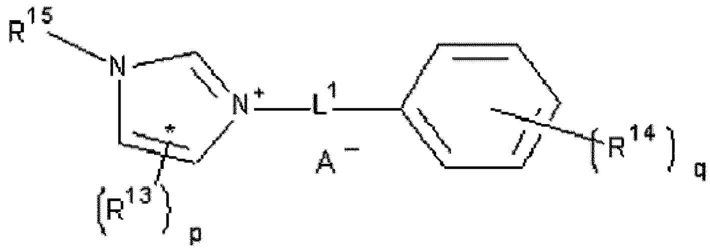

(Ib)

où

A est choisi dans l'ensemble constitué par [Br], [Cl], [$PF_6$], [$AsF_6$], [$SbF_6$], [$N(SO_2CF_3)_2$], [$BF_4$], [$CF_3SO_3$], [$CH_3CO_2$], [$CF_3SO_2$], [$NO_2$], [$NO_3$], [$ClO_4$], [I], et [$AlR^{10}_{4-n}R^{11}_n$] ;
$R^{10}$ est un halogène ou alkyle en $C_1$ à $C_{12}$ ; de préférence un halogène ou alkyle en $C_1$ à $C_6$ ;
$R^{11}$ est un halogène ; et
n est un entier choisi parmi 0, 1, 2, 3 et 4 ;
$L^1$ est un alkylène en $C_1$ à $C_6$ ;
chaque $R^{13}$ est indépendamment choisi parmi l'hydrogène, alkyle en $C_1$ à $C_6$ et alcoxy en $C_1$ à $C_6$ ;
chaque $R^{14}$ est indépendamment choisi dans l'ensemble constitué par halogéno, halogénoalkyle en $C_1$ à $C_6$, et halogénoalcoxy en $C_1$ à $C_6$ ;
$R^{15}$ est indépendamment choisi parmi l'hydrogène et alkyle en $C_1$ à $C_6$ ;
p est un entier choisi parmi 1, 2 et 3 ; et
q est un entier choisi parmi 1, 2 et 3.

4. Procédé pour effectuer une chromatographie de surface selon la revendication 3, dans lequel

A est choisi dans l'ensemble constitué par [Br], [Cl], [$PF_6$], [$AsF_6$], [$SbF_6$], [$BF_4$], [$CH_3CO_2$], [$CF_3SO_2$], [$NO_2$], [$NO_3$], [$ClO_4$], [I] ;
$L^1$ est un alkylène en $C_1$ à $C_4$ ;
chaque $R^{13}$ est indépendamment choisi parmi l'hydrogène, alkyle en $C_1$ à $C_4$ et alcoxy en $C_1$ à $C_4$, et est de préférence l'hydrogène ;
chaque $R^{14}$ est indépendamment choisi parmi chloro, fluoro et bromo, de préférence chloro et fluoro ;
$R^{15}$ est indépendamment choisi parmi l'hydrogène et alkyle en $C_1$ à $C_4$ ;
p vaut 1, 2 ou 3 ; et
q vaut 1, 2 ou 3.

5. Procédé pour effectuer une chromatographie de surface selon l'une quelconque des revendications 1 à 4, dans lequel le substrat comprend une résine à base de silice normale et à inversion de phases, éventuellement dérivée par des groupes alkyle ou des groupes aromatiques, de préférence le gel de silice 60F254.

6. Procédé pour effectuer une chromatographie de surface selon l'une quelconque des revendications 1 à 5, dans lequel la chromatographie est effectuée à une température de travail comprise entre 30°C et 250°C.

7. Procédé pour effectuer une chromatographie de surface selon l'une quelconque des revendications 1 à 6, dans lequel la chromatographie est effectuée avec un gradient de température.

**8.** Procédé pour effectuer une chromatographie de surface selon la revendication 7, dans lequel le gradient de température est supérieur ou égal à 1°C par minute.

**9.** Procédé pour effectuer une chromatographie de surface selon l'une quelconque des revendications 1 à 8, dans lequel la chromatographie est effectuée par utilisation d'une phase mobile choisie dans l'ensemble comprenant le toluène, le xylène, le tétrahydrofurane, un alcane en $C_4$ à $C_{20}$ ramifié ou non ramifié, le dioxyde de carbone supercritique, le trichlorobenzène, l'acétate d'éthyle, le diméthylsulfoxyde, le diméthylformamide, un liquide ionique de formule (I) ou (II) tel que défini dans l'une quelconque des revendications 1 et 2, et leurs mélanges ; éventuellement en présence d'un co-éluant choisi dans l'ensemble comprenant un alcool et un liquide ionique de formule (I) ou (II) tel que défini dans l'une quelconque des revendications 1 et 2.

**10.** Procédé pour effectuer une chromatographie de surface selon l'une quelconque des revendications 1 à 9, dans lequel la chromatographie est effectuée sous une pression externe par application soit i) d'un gaz neutre sous pression appliqué à une membrane sur la phase stationnaire plane ; soit ii) d'un coussin pneumatique ou d'une membrane métallique sur la phase stationnaire plane ; soit iii) d'une presse pneumatique sur la phase stationnaire plane.

**11.** Procédé pour effectuer une chromatographie de surface selon l'une quelconque des revendications 1 à 10, dans lequel la chromatographie est soumise à un gradient d'élution et comprend au moins deux étapes de chromatographie subséquentes avec des distances de migration progressivement décroissantes de la phase mobile sur la plaque de chromatographie plane, et dans lequel ledit gradient d'élution comprend une augmentation de la force d'élution de la phase mobile entre deux étapes de chromatographie subséquentes, lesdites étapes étant effectuées sur la même plaque de chromatographie plane.

**12.** Procédé pour effectuer une chromatographie de surface selon l'une quelconque des revendications 1 à 11, dans lequel la chromatographie de surface est choisie parmi une chromatographie sur couche mince (CCM), une chromatographie sur couche mince haute performance (CCM-HP), et une chromatographie sur couche surpressurisée (CCSP).

**13.** Procédé pour effectuer une chromatographie de surface selon l'une quelconque des revendications 1 à 12, dans lequel la chromatographie de surface est une chromatographie sur couche surpressurisée (CCSP).

**14.** Procédé pour effectuer une chromatographie de surface selon l'une quelconque des revendications 1 à 13, dans lequel le polymère est une polyoléfine.

**15.** Procédé pour effectuer une chromatographie de surface selon l'une quelconque des revendications 1 à 14, dans lequel le polymère est choisi parmi le polyéthylène, le polypropylène, le polystyrène, et le poly(acide lactique).

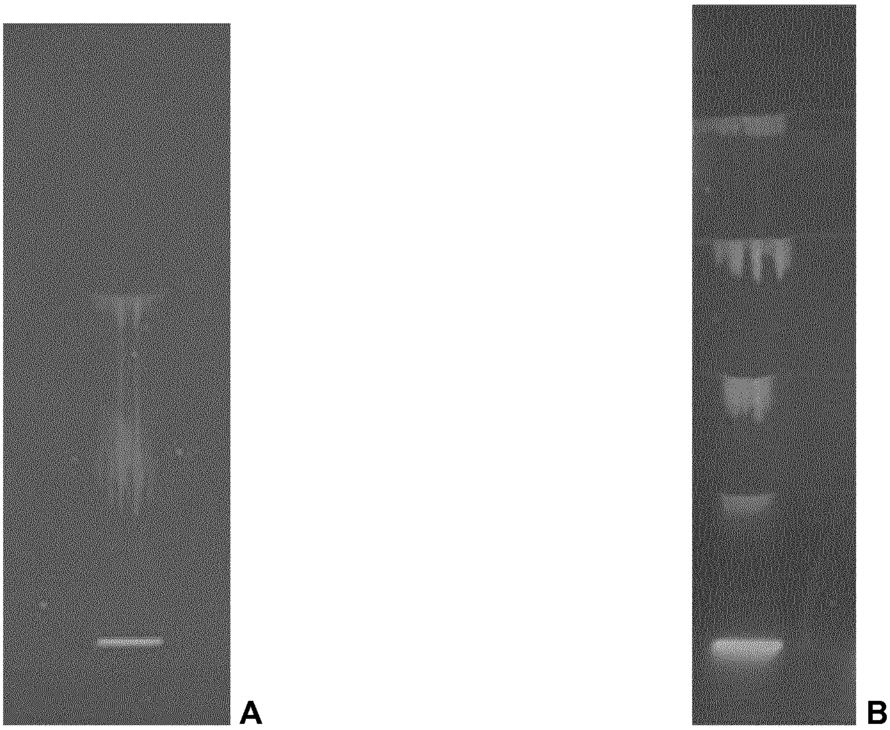


FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5994602 A
- WO 9618459 A
- WO 0181353 A
- US 20110178258 A


### Non-patent literature cited in the description


- **KLAUS K. UNGER.** Packings and Stationary Phases in Chromatographic Techniques. M. Decker, 1990